# EUROPEAN PATENT SPECIFICATION

(11) **EP 1 421 103 B1**
(45) Date of publication and mention of the grant of the patent: **15.10.2008**
(21) Application number: 02750842.3
(22) Date of filing: 18.07.2002
(51) Int. Cl.: C07K 1/00, C07K 14/605

(54) **METHOD FOR MAKING ACYLATED POLYPEPTIDES**
VERFAHREN ZUR HERSTELLUNG ACYLIERTER POLYPEPTIDE
PROCEDE DE PRODUCTION DE POLYPEPTIDES ACYLES

(30) Priority: 24.07.2001 DK 200101141
(43) Date of publication of application: 26.05.2004
(73) Proprietor: NOVO NORDISK A/S, 2880 Bagsvaerd (DK)
(72) Inventor: DIERS, Ivan, DK-3500 Vaerlose (DK); BALSCHMIDT, Per, DK-3060 Espergaerde (DK); MARKUSSEN, Jan, DK-2730 Herlev (DK); JONASSEN, Ib, DK-2500 Valby (DK); EGEL-MITANI, Michi, DK-2950 Vedbaek (DK); KJELDSEN, Thomas, Borglum, DK-2830 Virum (DK)
(74) Representative: Secher, Anne
(86) International application number: PCT/DK2002/000502
(87) International publication number: WO 2003/010186

(56) References cited:
- EP-A- 0 712 862
- WO-A-00/55119
- WO-A-98/28429
- US-A- 5 905 140
- US-A- 6 011 007

### SEQUENCE LISTING

<110> Novo Nordisk A/S
   Egel-Mitani, Michi
   Balscmidt, Per
   Markussen, Jan
   Diers, Ivan
   Borglum Kjeldsen, Thomas
   Jonassen, Ib
<120> Method for making acylated polypeptides
<130> 6289.204-WO
<160> 51
<170> PatentIn version 3.1
<210> 1
   <211> 5
   <212> PRT
   <213> Artificial Sequence
<220>
   <223> Synthetic peptide sequence
<400> 1
   Glu Glu Ala His Lys
   1 5
<210> 2
   <211> 7
   <212> PRT
   <213> Artificial Sequence
<220>
   <223> Artificial peptide
<400> 2
   Glu Glu Ala Glu Ala His Lys
   1 5
<210> 3
   <211> 9
   <212> PRT
   <213> Artificial Sequence
<220>
   <223> Artificial peptide
<400> 3
   Glu Glu Ala Glu Ala Glu Ala His Lys
   1 5
<210> 4
   <211> 5
   <212> PRT
   <213> Artificial Sequence
<220>
   <223> Artificial peptide
<400> 4
   Glu Glu Gly His Lys
   1 5
<210> 5
   <211> 4
   <212> PRT
   <213> Artificial Sequence
<220>
   <223> Artificial peptide
<400> 5
   Glu His Pro Lys
   1
<210> 6
   <211> 6
   <212> PRT
   <213> Artificial Sequence
<220>
   <223> Artificial peptide
<400> 6
   Glu Glu Gly Glu Pro Lys
   1 5
<210> 7
   <211> 5
   <212> PRT
   <213> Artificial Sequence
<220>
   <223> Artificial peptide
<400> 7
   Glu Glu His Cys Lys
   1 5
<210> 8
   <211> 5
   <212> PRT
   <213> Artificial Sequence
<220>
   <223> Artificial peptide
<400> 8
   Glu Glu His His Lys
   1 5
<210> 9
   <211> 5
   <212> PRT
   <213> Artificial Sequence
<220>
   <223> Artificial peptide
<400> 9
   Glu His His His Lys
   1 5
<210> 10
   <211> 5
   <212> PRT
   <213> Artificial Sequence
<220>
   <223> Artificial peptide
<400> 10
   Glu His Ala His Lys
   1 5
<210> 11
   <211> 5
   <212> PRT
   <213> Artificial Sequence
<220>
   <223> Artificial peptide
<400> 11
   Glu Gly Ala His Lys
   1 5
<210> 12
   <211> 6
   <212> PRT
   <213> Artificial Sequence
<220>
   <223> Artificial peptide
<400> 12
   Glu His Gly His Gly Lys
   1 5
<210> 13
   <211> 7
   <212> PRT
   <213> Artificial Sequence
<220>
   <223> Artificial peptide
<400> 13
   Glu Glu Ala His Glu Leu Lys
   1 5
<210> 14
   <211> 7
   <212> PRT
   <213> Artificial Sequence
<220>
   <223> Artificial peptide
<400> 14
   Glu Glu Ala His Glu Ile Lys
   1 5
<210> 15
   <211> 7
   <212> PRT
   <213> Artificial Sequence
<220>
   <223> Artificial peptide
<400> 15
   Glu Glu Ala His Glu Val Lys
   1 5
<210> 16
   <211> 7
   <212> PRT
   <213> Artificial Sequence
<220>
   <223> Artificial peptide
<400> 16
   Glu Glu Ala His Glu Met Lys
   1 5
<210> 17
   <211> 7
   <212> PRT
   <213> Artificial Sequence
<220>
   <223> Artificial peptide
<400> 17
   Glu Glu Ala His Glu Phe Lys
   1 5
<210> 18
   <211> 7
   <212> PRT
   <213> Artificial Sequence
<220>
   <223> Artificial peptide
<400> 18
   Glu Glu Ala His Glu Tyr Lys
   1 5
<210> 19
   <211> 7
   <212> PRT
   <213> Artificial Sequence
<220>
   <223> Artificial peptide
<400> 19
   Glu Glu Ala His Glu Trp Lys
   1 5
<210> 20
   <211> 8
   <212> PRT
   <213> Artificial Sequence
<220>
   <223> Artificial peptide
<400> 20
   Glu Glu Gly Asn Thr Thr Pro Lys
   1 5
<210> 21
   <211> 9
   <212> PRT
   <213> Artificial Sequence
<220>
   <223> Articicial peptide
<400> 21
   Glu Glu Gly Asn Glu Thr Glu Pro Lys
   1 5
<210> 22
   <211> 9
   <212> PRT
   <213> Artificial Sequence
<220>
   <223> Artificial peptide
<400> 22
   Glu Glu Gly Asn Asp Thr Glu Pro Lys
   1 5
<210> 23
   <211> 9
   <212> PRT
   <213> Artificial Sequence
<220>
   <223> Artificial peptide
<400> 23
   Glu Glu Gly Asn Thr Thr Glu Pro Lys
   1 5
<210> 24
   <211> 5
   <212> PRT
   <213> Artificial Sequence
<220>
   <223> Artificial peptide
<400> 24
   Gln Asp Ala His Lys
   1 5
<210> 25
   <211> 5
   <212> PRT
   <213> Artificial Sequence
<220>
   <223> Artificial peptide
<400> 25
   Gln Asp Thr Ala Lys
   1 5
<210> 26
   <211> 5
   <212> PRT
   <213> Artificial Sequence
<220>
   <223> Artificial peptide
<400> 26
   Asp Asp Asp Asp Lys
   1 5
<210> 27
   <211> 9
   <212> PRT
   <213> Artificial Sequence
<220>
   <223> Artificial peptide
<400> 27
   Glu Ala Glu Ala Trp His Trp Leu Lys
   1 5
<210> 28
   <211> 11
   <212> PRT
   <213> Artificial Sequence
<220>
   <223> Artificial peptide
<400> 28
   Glu Ala Glu Ala Glu Ala Trp His Trp Leu Lys
   1 5 10
<210> 29
   <211> 10
   <212> PRT
   <213> Artificial Sequence
<220>
   <223> Artificial peptide
<400> 29
   Glu Glu Ala Glu Ala Trp His Trp Leu Lys
   1               5                   10
<210> 30
   <211> 9
   <212> PRT
   <213> Artificial Sequence
<220>
   <223> Artificial peptide
<400> 30
   Glu Glu Glu Ala Trp His Trp Leu Lys
   1               5
<210> 31
   <211> 9
   <212> PRT
   <213> Artificial Sequence
<220>
   <223> Articicial peptide
<400> 31
   Leu Asp Gly Arg Leu Glu Ala Leu Lys
   1               5
<210> 32
   <211> 11
   <212> PRT
   <213> Artificial Sequence
<220>
   <223> Artificial peptide
<400> 32
   Glu Glu Leu Asp Gly Arg Leu Glu Ala Leu Lys
   1               5                   10
<210> 33
   <211> 11
   <212> PRT
   <213> Artificial Sequence
<220>
   <223> Artificial peptide
<400> 33
   Glu Glu Leu Asp Ala Arg Leu Glu Ala Leu Lys
   1 5 10
<210> 34
   <211> 4
   <212> PRT
   <213> Artificial sequence
<220>
   <223> Artificial peptide
<400> 34
   Asp Thr His Lys
   1
<210> 35
   <211> 4
   <212> PRT
   <213> Artificial sequence
<220>
   <223> Artificial peptide
<400> 35
   Asp Ala His Lys
   1
<210> 36
   <211> 7
   <212> PRT
   <213> Artificial Sequence
<220>
   <223> Synthetic peptide sequence
<400> 36
   Glu His His Gly His Gly Lys
   1 5
<210> 37
   <211> 4
   <212> PRT
   <213> Artificial Sequence
<220>
   <223> Synthetic peptide sequence
<400> 37
   Asp Ser His Lys
   1
<210> 38
   <211> 5
   <212> PRT
   <213> Artificial Sequence
<220>
   <223> Synthetic peptide sequence
<400> 38
   Gln Asp Thr His Lys
   1 5
<210> 39
   <211> 11
   <212> PRT
   <213> Artificial Sequence
<220>
   <223> Synthetic peptide sequence
<400> 39
   Glu Ala Glu Ala Glu Ala Gln Asp Thr His Lys
   1 5 10
<210> 40
   <211> 9
   <212> PRT
   <213> Artificial Sequence
<220>
   <223> Synthetic peptide sequence
<400> 40
   Glu Ala Glu Ala Gln Asp Thr His Lys
   1 5
<210> 41
   <211> 7
   <212> PRT
   <213> Artificial Sequence
<220>
   <223> Synthetic peptide sequence
<400> 41
   Glu Ala Gln Asp Thr His Lys
   1               5
<210> 42
   <211> 5
   <212> PRT
   <213> Artificial Sequence
<220>
   <223> Synthetic peptide sequence
<400> 42
   Trp His Trp Leu Lys
   1               5
<210> 43
   <211> 7
   <212> PRT
   <213> Artificial Sequence
<220>
   <223> Synthetic peptide sequence
<400> 43
   Glu Glu Trp His Trp Leu Lys
   1               5
<210> 44
   <211> 11
   <212> PRT
   <213> Artificial Sequence
<220>
   <223> Synthetic peptide sequence
<400> 44
   Glu Glu Glu Ala Glu Ala Trp His Trp Leu Lys
   1 5 10
<210> 45
   <211> 5
   <212> PRT
   <213> Artificial Sequence
<220>
   <223> Synthetic peptide sequence
<400> 45
   Glu Glu Ala Glu Lys
   1 5
<210> 46
   <211> 6
   <212> PRT
   <213> Artificial Sequence
<220>
   <223> Synthetic peptide sequence
<400> 46
   Glu Glu Gly Glu Pro Lys
   1 5
<210> 47
   <211> 7
   <212> PRT
   <213> Artificial Sequence
<220>
   <223> Synthetic peptide sequence
<400> 47
   Glu Ala Gln Asp Ala His Lys
   1 5
<210> 48
   <211> 9
   <212> PRT
   <213> Artificial Sequence
<220>
   <223> Synthetic peptide sequence
<400> 48
   Glu Ala Glu Ala Gln Asp Ala His Lys
   1               5
<210> 49
   <211> 11
   <212> PRT
   <213> Artificial Sequence
<220>
   <223> Synthetic peptide sequence
<400> 49
   Glu Ala Glu Ala Glu Ala Gln Asp Ala His Lys
   1               5                   10
<210> 50
   <211> 4
   <212> PRT
   <213> Artificial Sequence
<220>
   <223> Synthetic peptide sequence
<220>
   <221> misc_feature
   <222> (1)..(4)
   <223> Xaa can be Ala , Thr or Ser
<400> 50
   Asp Xaa His Lys
   1
<210> 51
   <211> 4
   <212> PRT
   <213> Artificial Sequence
<220>
   <223> Synthetic peptide sequence
<220>
   <221> misc_feature
   <222> (1)..(2)
   <223> Xaa can be Glu, Asp, Asn, Gln, Thr, Gly, Leu, Ile, or Tyr
<220>
   <221> misc_feature
   <222> (2)..(3)
   <223> Xaa can be Leu, Ile, Val, Met, Phe, Tyr, Trp or Cys
<400> 51
   His Xaa Xaa Lys
   1

## Description

### FIELD OF THE INVENTION

The present invention is related to a method of producing polypeptides in transformed host cells by expressing a precursor molecule of the desired polypeptide which is to be acylated and subsequently cleaved at a Lys cleavage site in a subsequent in vitro step. The invention is also related to DNA-sequences, vectors and transformed host cells for use in the claimed method. Further, the present invention is related to certain precursors of the desired polypeptides and certain acylation methods.

### BACKGROUND OF THE INVENTION

Recombinant DNA technology has enabled expression of foreign (heterologous) polypeptides in microbial and other host cells. In yeast expression of heterologous polypeptides after transformation of yeast cells with suitable expression vectors comprising DNA sequences coding for said polypeptides has been successful for many species of polypeptides, such as insulin and insulin precursors, glucagon, glucagon like peptides and analogues thereof.

A common problem with expression of proteins or polypeptides of a limited size in a recombinant host is, however, proteolytic degradation of the expressed product by proteolytic enzymes produced by the host organism.

Thus, the isolated product may be a heterogeneous mixture of species of the desired polypeptide having different amino acid chain lengths. Another problem encountered in production of heterologous polypeptides in yeast may be low yield, presumably due to proteolytic processing both in intracellular compartments and at the plasma membrane caused by aberrant processing at internal sites in the polypeptide. Yeast contains a number of proteases used for processing yeast proteins e.g. Kex2p and Yps1p which cleave at the C-terminal side of a dibasic amino acid sequence, and the carboxypeptidase Kex1p which digests remaining basic amino acids after the endoproteolytic digestion by Kex2p, and Ste13p or Dap2p which cleave at X-Ala or X-Pro.

Some polypeptides, e.g. polypeptides having from about 10 to about 100 amino acids chains and none or a few disulphide bonds and/or are rich in basic amino acids, such as β-endorphine, glucagon and glucagon like peptides may be especially susceptible to intracellular and extracellular proteolytic degradation when expressed in a transformed host cell due to their short-chain open and non-disulfide stabilized structure resulting in an inhomogeneous product which may be proteolytically degraded in the N- and C-terminal ends as well as endoproteolytically degraded.

Furthermore, N-terminal cleavage of expressed polypeptides by host cell produced enzymes may cause decreased yield of a desired product with correct N-terminal if the N-terminal of the expressed product constitutes a cleavage site for endogenous enzymes. In yeast for example the enzyme Ste13p cleaves at X-Ala or X-Pro, where X can be any amino acid residue. Thus, polypeptides with an Ala or Pro residue as the second residue from the N-terminal end may be cleaved at the N-terminal end and the recovered polypeptide may be a mixture of different degradation products complicating the recovery process and reducing the overall yield.

Furthermore, small polypeptides with no or little tertiary structure and low content of α-helixes may have a higher tendency to form β-sheets that stack on each other and form fibrils during fermentation and down stream separation and purification steps in large scale production. Formation of fibrils may cause unwanted precipitation with loss of the desired product. Fibrillation may be prevented by treatment at high pH. However, such alkaline treatment is pretty harsh to the product and may cause unwanted formation of D-amino acids residues.

Human GLP-1 is a 37 amino acid residue peptide originating from preproglucagon which is synthesised in the L-cells in the distal ileum, in the pancreas and in the brain. Processing of preproglucagon to give GLP-1₍₇₋₃₆₎amide, GLP-1₍₇₋₃₇) and GLP-2 occurs mainly in the L-cells. Both GLP-1 and GLP-2 has an Ala as the second amino acid residue from the N-terminal end and are thus prone for N-terminal cleavage when expressed in a host organism such as yeast.

Introduction of lipophilic acyl groups in naturally occurring peptides or analogues thereof has shown to lead to acylated peptides which have a protracted profile relative to the native peptide or unmodified analogues. This phenomenon is disclosed and demonstrated in WO 98/08871 which discloses acylation of GLP-1 and analogues thereof and in WO 98/08872 which discloses acylation of GLP-2 and analogues thereof.

US patent No. 6,011,007 discloses a method for acylating an N-terminally extended insulin precursor at the ε-NH₂ of LysB29 and subsequently hydrolysing the Arg bond between B1 and the N-terminal extension with trypsin and carboxypeptidase B. WO 0055119 discloses an acylation method where an free amino group is reacted with an activated amid at basic pH, e.g. acylation of Arg³⁴GLP1⁷⁻³⁷ at the ε-NH₂ group of Lys²⁶. WO 9535384 discloses production of recombinant proteins in yeast where the proteins are expressed with an N-terminal extension which may later on be cleaved off.

When acylating precursor molecules comprising a Lys cleavage site acylation of the Lys cleavage site should be avoided as such acylation will prevent the subsequent cleavage. The method according to the present invention is a solution to this problem as it will appear from the following.

### SUMMARY OF THE INVENTION

In its broadest aspect, the present invention is related to a method of producing polypeptides in transformed host cells by expressing a precursor molecule of the desired polypeptide said precursor molecule comprising an N-terminal extension which allows for preferential acylation of the expressed precursor molecule and protects the expressed precursor molecule against proteolytic degradation within the host cell or in the culture medium. In addition, the precursor molecule is easier to purify and has a less tendency to form fibrils thus allowing more flexibility when selecting down stream separation and purification steps in large scale operations.

In one aspect the present invention is related to a method for making a polypeptide comprising at least one lysine residue being acylated in its ε-amino group, said method comprising the following steps:
(i) culturing a yeast cell comprising a polynucleotide sequence encoding a precursor molecule of the desired polypeptide under suitable conditions for expression of said precursor molecule, the precursor molecule comprising the desired polypeptide and an N-terminal extension, said N-terminal extension being cleavable from the desired polypeptide at a lysine cleavage site and having the following formula:

   Xₙ------X₁ - Lys

   wherein Lys is a cleavage site and Xₙ-----X₁ is a peptide sequence of from 2-14 amino acid residues in length having the function of preventing or minimizing acylation of the free ε-amino group in the Lys cleavage site in step (iii) and having the further function of protecting the expressed precursor polypeptide from endoproteolytic cleavage with the proviso that no X is Lys and that at least one X is His or Glu or Asp,
(ii) separating the expressed precursor molecule from the culture broth;
(iii) preferentially acylating the ε-amino group of at least one lysine in the desired polypeptide without acylating the ε-amino group of the Lys-cleavage site in the N-terminal extension;
(iv) removing the N-terminal extension from the acylated precursor molecule by enzymatic cleavage and
(v) isolating the acylated polypeptide by suitable means.

### DETAILED DESCRIPTION OF THE INVENTION

The N-terminal extension will typically be up to 15 amino acids in length and may be from 3-15; 3-12; 3-10; 3-9; 3-8; 3-7; 3-6; or 3-5 amino acids in length. The amino acids in the N-terminal extension are selected with a multiple purpose: 1) to prevent or minimize acylation of the Lys cleavage site at the C-terminal end of the N-terminal extension; 2) to protect the expressed precursor molecule against endoproteolytic degradation; and 3) to prevent precipitation caused by fibrillation during fermentation and down stream processing steps such as separation and purification in large scale production. Furthermore, the amino acid residues at both ends of the N-terminal extension should be selected so as to ensure efficient cleavage of the N-terminal extension from the desired polypeptide at the C-terminal end (at the Lys-cleavage site) and in the yeast cell at the N-terminal end from possible upstream sequences such as pre- or pre-pro peptides which have the purpose of ensuring transport of the expressed polypeptide out of the host cell and into the culture medium. Finally, the N-terminal extension may serve as a tag for purification purposes.

In one embodiment the present invention is related to a method, wherein one or more amino acid residues in the N-terminal extension are capable of establishing a metal ion complex binding site together with one or more amino acid residues in the N-terminal end of the polypeptide.

The N-terminal extension comprising a metal ion binding site may be derived from the N-terminal of albumins. Examples of Cu⁺² binding sites are the N-terminals of bovine (Asp-Thr-His-Lys,SEQ ID NO:34) and human (Asp-Ala-His-Lys, SEQ ID NO:35) serum albumin (Peters, T.; All about Albumin, Academic Press, USA (1996) p 121-123) which could be used as the N-terminal extension in synergy with heavy metal ions such as Cu⁺⁺ present in the culture medium and during the acylation step (iii).

The N-terminal extension comprising a metal ion binding site may also be derived from the Zn-binding sites in especially some of the metalloendopeptidases EC 3.4.24-.

In one embodiment, the N-terminal extension comprises at least one histidine residue. The histidine residues in the N-terminal extension are typically positioned 1 - 4 residues from the lysine cleavage site.

Examples of N-terminal extensions comprising at least one histidine residue are Glu-Glu-Ala-His-Lys( SEQ ID NO:1); Glu-(Glu-Ala)₂-His-Lys(SEQ ID NO:2); Glu-(Glu-Ala)₃His-Lys(SEQ ID NO:3); Glu-Glu-Gly-His-Lys(SEQ ID NO:4); Glu-His-Pro-Lys(SEQ ID NO:5); Glu-Glu-Gly-Glu-Pro-Lys(SEQ ID NO:6); Glu-Glu-His-Cys-Lys(SEQ ID NO:7); Glu-Glu-His-His-Lys(SEQ ID NO:8); Glu-His-His-His-Lys(SEQ ID NO:9); Glu-His-Ala-His-Lys(SEQ ID NO:10); Glu-Gly-Ala-His-Lys(SEQ ID NO:11); Glu-His-Gly-His-Gly-Lys(SEQ ID NO:12); Glu-Glu-Ala-His-Glu-Leu-Lys(SEQ ID NO:13); Glu-Glu-Ala-His-Glu-Ile-Lys(SEQ ID NO:14); Glu-Glu-Ala-His-Glu-Val-Lys(SEQ ID NO:15); Glu-Glu-Ala-His-Glu-Met-Lys(SEQ ID NO:16); Glu-Glu-Ala-His-Glu-Phe-Lys(SEQ ID NO:17); Glu-Glu-Ala-His-Glu-Tyr-Lys(SEQ ID NO:18); Glu-Glu-Ala-His-Glu-Trp-Lys(SEQ ID NO:19); Gln-Asp-Ala-His-Lys(SEQ ID NO:24); Glu-Glu-Glu-Ala-Trp-His-Trp-Leu-Lys(SEQ ID NO:30); Asp-Thr-His-Lys(SEQ ID NO:34); Glu-His-His-Gly-His-Gly-Lys(SEQ ID NO:36); Asp-Ser-His-Lys(SEQ ID NO:37); Gln-Asp-Thr-His-Lys(SEQ ID NO:38); Glu-Ala-Glu-Ala-Glu-Ala-Gln-Asp-Thr-His-Lys(SEQ ID NO:39); Glu-Ala-Glu-Ala-Gln-Asp-Thr-His-Lys( SEQ ID NO:40); Glu-Ala-Gln-Asp-Thr-His-Lys(SEQ ID NO:41); Trp-His-Trp-Leu-Lys(SEQ ID NO:42); Glu-Glu-Trp-His-Trp-Leu-Lys(SEQ ID NO:43); Glu-Glu-Glu-Ala-Glu-Ala-Trp-His-Trp-Leu-Lys(SEQ ID NO:44); Glu-Ala-Gln-Asp-Ala-His-Lys(SEQ ID NO:47); Glu-Ala-Glu-Ala-Gln-Asp-Ala-His-Lys(SEQ ID NO:48); and Glu-Ala-Glu-Ala-Glu-Ala-Gln-Asp-Ala-His-Lys(SEQ ID NO:49).

In another embodiment the present invention is related to a method, wherein the N-terminal extension of the precursor molecule comprises at least one amino acid residue that is capable of establishing a salt bridge (ion bond) with the lysine cleavage site N-terminal to the polypeptide.

In this embodiment, the N-terminal extension will typically comprise at least one Glu or Asp which may be positioned between 1 to 5 residues from the lysine cleavage site. In one such embodiment, the N-terminal extension comprises a Glu-Glu-sequence. Examples of N-terminal extensions comprising a Glu residue are Glu-Glu-Ala-Glu-Lys(SEQ ID NO:45); Glu-Glu-Gly-Glu-Pro-Lys(SEQ ID NO:46) and Glu-Glu-Lys. In this embodiment, the N-terminal extension may furthermore be capable of forming an α-helix.

N-terminal extensions functioning both as a tag and being capable of forming a salt bridge are sequences derived from the enterokinase site in bovine trypsinogen that is known to bind Ca⁺². Thus a suitable N-terminal extension for use in the present process is Asp-Asp-Asp-Asp-Lys(SEQ ID NO:26).

An N-terminal extension which is capable of forming an α-helix will typically comprise a sequence that contains alternating polar and non-polar amino acids as described by Zhu et al, Protein Science 2, 384-394 (1993). Examples of such sequences are Glu-Glu-Ala-Glu-Ala-Trp-His-Trp-Leu-Lys(SEQ ID NO:29), Glu-Glu-Glu-Ala-Trp-His-Trp-Leu-Lys(SEQ ID NO:30); Leu-Asp-Gly-Arg-Leu-Glu-Ala-Leu-Lys(SEQ ID NO:31); Glu-Glu-Leu-Asp-Gly-Arg-Leu-Glu-Ala-Leu-Lys(SEQ ID NO:32); Glu-Glu-Leu-Asp-Ala-Arg-Leu-Glu-Ala-Leu-Lys(SEQ ID NO:33); Glu-Glu-Trp-His-Trp-Leu-Lys(SEQ ID NO:43); and Glu-Glu-Glu-Ala-Glu-Ala-Trp-His-Trp-Leu-Lys(SEQ ID NO:44).

In this embodiment, the N-terminal extension may also comprise a eukaryotic N-glycosylation site N-X-S or N-X-T where X can be any amino acid residue except Pro. Examples of N-terminal extensions comprising a glycosylation site are Glu-Glu-Gly-Asn-Thr-Thr-Pro-Lys(SEQ ID NO:20), Glu-Glu-Gly-Asn-Glu-Thr-Glu-Pro-Lys(SEQ ID NO:21), Glu-Glu-Gly-Asn-Asp-Thr-Glu-Pro-Lys(SEQ ID NO:22) and Glu-Glu-Gly-Asn-Thr-Thr-Glu-Pro-Lys (SEQ ID NO: 23).

In a further embodiment the present invention is related to a method, wherein the desired polypeptide is sensitive to proteolytic degradation at its N-terminal end and wherein the N-terminal extension prevents or minimizes such proteolytic degradation. Such polypeptides may be characterized by having an Ala, Ser, Pro or Gly residue as the second amino acid residue from the N-terminal end.

In a still further embodiment the polypeptide has a His or Tyr as the N-terminal amino acid residue.

The N-terminal extension has the formula

Xₙ-----X₁ - Lys

wherein Lys is a cleavage site and Xₙ------X₁ is a peptide sequence of from 2-14 amino acid residues in length having the function of preventing or minimizing that the free ε-amino group in the Lys cleavage site will be acylated in the above described step (iii). Xₙ-X₁ will furthermore protect the expressed desired polypeptide from endoproteolytic cleavage and will prevent precipitation caused by fibrillation of the N-terminal extended molecule during fermentation and down stream separation and purification steps. The amino acid residues in Xₙ-----X₁ are furthermore selected so that optimal cleavage of the N-terminal extension at its C-terminal end (at Lys) is achieved. Furthermore, the amino acid residues in the N-terminal end of the extension are chosen so that cleavage is optimized in the yeast cell from upstream signal-leader-sequences at a KEX cleavage site (Lys,Arg). The amino acid residues in Xₙ-----X₁ may in principle be any amino acid residue except Lys as long as the peptide sequence fulfils at least one of the required purposes with the proviso that at least one X is His or Glu or Asp and that number two amino acid residue from the N-terminal end of the extension is preferably not Ala or Pro.

In one embodiment Xₙ------X₁ is of 2-12 amino acid residues in length. In another embodiment Xₙ------X₁ is of 2-10; 2-9; 2-8; 2-7; 2-6; or 2-5 amino acid residues in length.

In another embodiment Xₙ------X₁ contains 2-8 amino acid residues which are selected from the group consisting of His; Glu; Ala; Asp; Gly; and Pro.

In another embodiment Xₙ------X₁ contains 4-10 amino acid residues which are selected from the group consisting of Glu; Asp; Ala; His; Trp; Tyr; Ile; Val; Met; and Phe.

In a further embodiment Xₙ------X₁ contains 5 - 8 amino acid residues selected from the group consisting of Glu; Asp; Gly; Asn; Thr; Ser; and Pro.

In all embodiments Glu and/or Asp are preferably selected as the first and second amino acid residue from the N-terminal end of the extension to ensure proper cleavage at this end from an upstream pre- or pre-pro-sequence by means of a Kex2p cleavage site. Furthermore, one or more Glu and/Asp residues at the N-terminal end of the N-terminal extension will protect the N-terminal end of the ultimately desired polypeptide from in vivo degradation during fermentation.

Examples of Xₙ------X₁ - Lys sequences are Glu-Glu-Ala-His-Lys(SEQ ID NO:1); Glu-(Glu-Ala)₂-His-Lys(SEQ ID NO:2); Glu-(Glu-Ala)₃His-Lys(SEQ ID NO:3); Glu-Glu-Gly-His-Lys( SEQ ID NO:4); Glu-His-Pro-Lys(SEQ ID NO:5); Glu-Glu-Gly-Glu-Pro-Lys(SEQ ID NO:6); Glu-Glu-His-Cys-Lys(SEQ ID NO:7); Glu-Glu-His-His-Lys(SEQ ID NO:8); Glu-His-His-His-Lys(SEQ ID NO:9); Glu-His-Ala-His-Lys(SEQ ID NO:10); Glu-Gly-Ala-His-Lys(SEQ ID NO:11); Glu-His-Gly-His-Gly-Lys(SEQ ID NO:12); Glu-Glu-Ala-His-Glu-Leu-Lys(SEQ ID NO:13); Glu-Glu-Ala-His-Glu-Ile-Lys(SEQ ID NO:14); Glu-Glu-Ala-His-Glu-Val-Lys(SEQ ID NO:15); Glu-Glu-Ala-His-Glu-Met-Lys(SEQ ID NO:16); Glu-Glu-Ala-His-Glu-Phe-Lys(SEQ ID NO:17); Glu-Glu-Ala-His-Glu-Tyr-Lys(SEQ ID NO:18); Glu-Glu-Ala-His-Glu-Trp-Lys(SEQ ID NO:19); Glu-Glu-Gly-Asn-Thr-Thr-Pro-Lys(SEQ ID NO:20); Glu-Glu-Gly-Asn-Glu-Thr-Glu-Pro-Lys(SEQ ID NO:21), Glu-Glu-Gly-Asn-Asp-Thr-Glu-Pro-Lys(SEQ ID NO:22); Glu-Glu-Gly-Asn-Thr-Thr-Glu-Pro-Lys(SEQ ID NO: 23); Gln-Asp-Ala-His-Lys(SEQ ID NO:24); Glu-Glu-Lys; Asp-Asp-Asp-Asp-Lys(SEQ ID NO:26); Glu-Glu-Ala-Glu-Ala-Trp-His-Trp-Leu-Lys(SEQ ID NO:29); Glu-Glu-Glu-Ala-Trp-His-Trp-Leu-Lys(SEQ ID NO:30); Leu-Asp-Gly-Arg-Leu-Glu-Ala-Leu-Lys(SEQ ID NO:31); Glu-Glu-Leu-Asp-Gly-Arg-Leu-Glu-Ala-Leu-Lys (SEQ ID NO:32); Glu-Glu-Leu-Asp-Ala-Arg-Leu-Glu-Ala-Leu-Lys(SEQ ID NO:33); Asp-Thr-His-Lys(SEQ ID NO:34); Asp-Ala-His-Lys(SEQ ID NO:35); Glu-His-His-Gly-His-Gly-Lys(SEQ ID NO:36); Asp-Ser-His-Lys(SEQ ID NO:37); Gln-Asp-Thr-His-Lys(SEQ ID NO:38); Glu-Ala-Glu-Ala-Glu-Ala-Gln-Asp-Thr-His-Lys(SEQ ID NO:39); Glu-Ala-Glu-Ala-Gln-Asp-Thr-His-Lys(SEQ ID NO:40); Glu-Ala-Gln-Asp-Thr-His-Lys(SEQ ID NO:41) Trp-His-Trp-Leu-Lys(SEQ ID NO:42); Glu-Glu-Trp-His-Trp-Leu-Lys(SEQ ID NO:43); Glu-Glu-Glu-Ala-Trp-His-Trp-Leu-Lys(SEQ ID NO:44); Glu-Glu-Ala-Glu-Lys(SEQ ID NO:45); Glu-Glu-Gly-Glu-Pro-Lys(SEQ ID NO:46); Glu-Ala-Gln-Asp-Ala-His-Lys(SEQ ID NO:47); Glu-Ala-Glu-Ala-Gln-Asp-Ala-His-Lys(SEQ ID NO:48); Glu-Ala-Glu-Ala-Glu-Ala-Gln-Asp-Ala-His-Lys(SEQ ID NO:49);

In a further embodiment, the N-terminal extension has the sequence Asp-X-His-Lys(SEQ ID NO:50) where X is Ala, Thr or Ser. The sequence Asp-X-His-Lys constitutes a heavy metal ion albumin binding site.

In a still further embodiment, the N-terminal extension has the sequence His - Z₁ -Z₂ - Lys(SEQ ID NO:51) wherein Z₁ is Glu; Asp; Asn; Gln; Ser; Thr; Gly; Leu; Ile; Val; Met; Phe or Tyr and Z₂ is Leu; Ile; Val; Met; Phe; Tyr; Trp or Cys. In one embodiment Z₁ is Glu or Asp. The sequence His-Z₁-Z₂ -Lys will together with an N-terminal His residue in the desired polypeptide constitute a metal binding site homologue to Zn-binding sites in certain metalloendopeptidases such as Glu-Glu-Ala-His-Glu-Leu-Lys(SEQ ID NO:13); Glu-Glu-Ala-His-Glu-Ile-Lys(SEQ ID NO:14); Glu-Glu-Ala-His-Glu-Val-Lys(SEQ ID NO:15); Glu-Glu-Ala-His-Glu-Met-Lys(SEQ ID NO:16); Glu-Glu-Ala-His-Glu-Phe-Lys(SEQ ID NO:17); Glu-Glu-Ala-His-Glu-Tyr-Lys(SEQ ID NO:18); and Glu-Glu-Ala-His-Glu-Trp-Lys(SEQ ID NO:19).

The N-terminal extension is found to be stably attached to the precursor molecule of the invention during fermentation, protecting the N-terminal end of the precursor molecule against the proteolytic activity of yeast proteases such as Ste13p or Dap2p.

The N-terminal extension will be removed from the acylated recovered precursor molecule by means of a proteolytic enzyme which is specific for Lys. Examples of such proteolytic enzymes are trypsin or *Achromobacter lyticus* protease I.

The polypeptide precursor for the desired polypeptide may have the formula

N-terminal-extension-Lys - Z₃ - Z₄ - *polypeptide*

wherein Lys is a cleavage site, the N-terminal extension has 2-14 amino acid residues as described above, Z₃ is the N-terminal amino acid residue in the desired polypeptide and is His or Tyr, Z₄ is the next amino acid residue from the N-terminal end in the desired polypeptide and is Ala, Ser or Gly, and *polypeptide* is the remaining sequence of the desired polypeptide.

In one embodiment of the present invention *polypeptide * is the relevant portion of GLP-1 or GLP-2.

Introduction of lipophilic acyl groups in naturally occurring peptides or analogues thereof has shown to lead to acylated peptides which have a protracted profile relative to the native peptide or unmodified analogues. This phenomenon is disclosed and demonstrated in WO 98/08871 which discloses acylation of GLP-1 and analogues thereof and in WO 98/08872 which discloses acylation of GLP-2 and analogues thereof. The lipophilic group may be introduced by means of mono- or dipeptide spacers as disclosed in WO 98/08871. Alternatively, the lipophilic group may be introduced by means of α-amino-α,ω-dicarboxylic acid groups as disclosed in WO 00/55119.

The present polypeptide precursor molecules will contain at least two lysine groups with a free ε-amino group, i.e. the lysine cleavage residue and at least one lysine residue in the desired polypeptide. If all lysine groups were acylated including the lysine cleavage residue, subsequent cleavage of the N-terminal extension from the desired polypeptide sequence will not be possible. Thus one of the purposes to express the desired polypeptide with an N-terminal extension is to prevent or minimize acylation of the free ε-amino group in the lysine cleavage site. The precursor molecule can then be preferentially acylated in the desired lysine residue which in the case of GLP-1 is the lysine in position 26. After acylation the acylated precursor molecule is cleaved by suitable enzymatic means as described above and the desired acylated polypeptide can be isolated.

The acylation step (iii) may be conducted at a pH between 7 and 12. In certain embodiments, the pH will be between 8 and 11.5 or between 9.0 and 10.5 and a pH value of about 9.5 to 10.5 has proven to be efficient. The temperature will be between minus 5 and 35°C and will typically be between 0 and 20°C or between 15 and 30°C.

In one embodiment of the present invention the acylation is conducted in the presence of divalent metal ions such as Cu²⁺, Fe²⁺, Ni²⁺, Co²⁺, Zn²⁺, Mg²⁺, Mn²⁺ and Ca²⁺. However, trivalent metal ions such as Co³⁺ are also efficient for the purpose of the present invention.

According to further aspects the present invention is related to polynucleotides encoding the claimed polypeptide precursors and vectors and transformed host cells containing such polynucleotides.

### DEFINITIONS

The term "**preferential acylating**" is meant to include and acylation process where acylation takes place at one or more preferred positions in the molecule in a substantial higher degree than at other positions in the same molecule. Thus, the acylation at the preferred positions is at least 50%, preferably at least 80% and most preferred 90-100% of the total acylation. In the present method acylation of the ε-amino group of the lysine cleavage site in the N-terminal extension should be avoided or minimized as much as possible as acylation at this position may interfere with the subsequent cleavage of the N-terminal extension from the desired end product leading to yield loss.

With "**N-terminal extension**" is meant a polypeptide sequence removably attached to the N-terminal amino acid residue in the desired polypeptide. The N-terminal extension may be 2-15 amino acid residues in length and will comprise a Lys residue as its C-terminal amino acid residue for cleavage from the desired polypeptide. The N-terminal extension will protect the expressed fusion polypeptide against proteolytic degradation within the host cell as described above. In addition, it is believed to prevent or minimize acylation of the ε-amino group of the lysine cleavage site in the N-terminal extension by masking said Lys residue during the acylation process.

With "**desired polypeptide**" is meant the ultimate polypeptide obtained after cleavage of the N-terminal extension from the precursor molecule. This expression will cover both the acylated and non-acylated version of said polypeptide. The "**N-terminal extension**" includes the lysine residue which constitutes the cleavage site for cleavage of the N-terminal extension from the desired polypeptide's N-terminal end. It will be understood that whenever a Lys-group is shown as the C-terminal amino acid of an N-terminal extension or a sequence being comprised in the N-terminal extension, then said Lys-residue is directly linked to the N-terminal amino acid residue of the desired polypeptide and constitutes the cleavage site. Cleavage at the Lys-residue (step iv) is preferably accomplished by means of a proteolytic enzyme which is specific Lys. Examples of such proteolytic enzymes are trypsin or *Achromobacter lyticus* protease (ALP).

An example of a desired polypeptide is GLP-1. The amino acid sequence of GLP-1 is given *i.a.* by Schmidt et al. (Diabetologia 28 704-707 (1985). Although the interesting pharmacological properties of GLP-1(7-37) and analogues thereof have attracted much attention in recent years only little is known about the structure of these molecules. The secondary structure of GLP-1 in micelles has been described by Thorton et al. (Biochemistry 33 3532-3539 (1994)), but in normal solution,GLP-1 is considered a very flexible molecule.

A simple system is used to describe fragments and analogues of this peptide. Thus, for example, Gly⁸GLP-1₍₇₋₃₇) designates a fragment of GLP-1 derived from GLP-1₍₁₋₃₇₎ by deleting the amino acid residues Nos. 1 to 6 and substituting the naturally occurring amino acid residue in position 8 (Ala) by Gly. Similarly, Lys²⁶(N^{ε}-tetradecanoyl)-GLP-1₍₇₋₃₇₎ designates GLP-1₍₇₋₃₇₎ wherein the ε-amino group of the Lys residue in position 26 has been tetradecanoylated.

Other examples of a desired polypeptides are GLP-2 and glucagon both belonging to the GRF (growth hormone releasing factor) family of peptides having a His or Tyr in the N-terminal position and Ser, Ala or Gly in the next position, vide Adelhorst K. et al., The Journal of Biological Chemistry (1994) p 6275 -6278).

*"**POT**"* is the *Schizosaccharomyces pombe* triose phosphate isomerase gene, and *"**TPI1**"* is the *S. cerevisiae* triose phosphate isomerase gene.

With "**fibrillation**" is meant a process where so called "**fibrils**" are formed. "**Fibrils**" is a well recognized and described phenomenon and may be composed of antiparallel β-sheets. Molecules like GLP's with little α-helical structure and a very flexible and little tertiary structure are very prone to aggregation that leads to precipitation and loss of yield if very crude chemical conditions are not taken in use such as alkaline treatment at pH ∼12.

By a "**leader**" is meant an amino acid sequence consisting of a pre-peptide (the signal peptide) and a pro-peptide.

The term "**signal peptide**" is understood to mean a pre-peptide which is present as an N-terminal sequence on the precursor form of a protein. The function of the signal peptide is to allow the heterologous protein to facilitate translocation into the endoplasmic reticulum. The signal peptide is normally cleaved off in the course of this process. The signal peptide may be heterologous or homologous to the yeast organism producing the protein. A number of signal peptides may be used with the DNA construct of the invention including the YPS1signal peptide (formally called the YAP3 signal peptide) or any functional analogue thereof (Egel-Mitani et al. (1990) YEAST 6:127-137 and US 5,726,038) and the α-factor signal of the *MFα1* gene (Thorner (1981) in The Molecular Biology of the Yeast Saccharomyces cerevisiae, Strathern et al., eds., pp 143-180, Cold Spring Harbor Laboratory, NY and US 4,870,00.

The term "**pro-peptide**" means a polypeptide sequence whose function is to allow the expressed polypeptide to be directed from the endoplasmic reticulum to the Golgi apparatus and further to a secretory vesicle for secretion into the culture medium (i.e. exportation of the polypeptide across the cell wall or at least through the cellular membrane into the periplasmic space of the yeast cell). The pro-peptide may be the yeast α-factor pro-peptide, vide US 4,546,082 and 4,870,008. Alternatively, the pro-peptide may be a synthetic pro-peptide, which is to say a pro-peptide not found in nature. Suitable synthetic pro-peptides are those disclosed in US 5,395,922; 5,795,746; 5,162,498 and WO 98/32867. The pro- peptide will preferably contain an endopeptidase processing site at the C-terminal end, such as a Lys-Arg sequence or any functional analog thereof.

The polynucleotide sequence of the invention may be prepared synthetically by established standard methods, e.g. the phosphoamidite method described by Beaucage et al. (1981) Tetrahedron Letters 22:1859-1869, or the method described by Matthes et al. (1984) EMBO Journal 3:801-805. According to the phosphoamidite method, oligonucleotides are synthesized, for example, in an automatic DNA synthesizer, purified, duplexed and ligated to form the synthetic DNA construct. A currently preferred way of preparing the DNA construct is by polymerase chain reaction (PCR).

The polynucleotide sequence of the invention may also be of mixed genomic, cDNA, and synthetic origin. For example, a genomic or cDNA sequence encoding a leader peptide may be joined to a genomic or cDNA sequence encoding the precursor molecule of the invention, after which the DNA sequence may be modified at a site by inserting synthetic oligonucleotides encoding the desired amino acid sequence for homologous recombination in accordance with well-known procedures or preferably generating the desired sequence by PCR using suitable oligonucleotides.

The invention encompasses a vector which is capable of replicating in the selected microorganism or host cell and which carries a polynucleotide sequence encoding the precursor molecule of the invention. The recombinant vector may be an autonomously replicating vector, *i.e.,* a vector which exists as an extra-chromosomal entity, the replication of which is independent of chromosomal replication, *e.g.*, a plasmid, an extra-chromosomal element, a mini-chromosome, or an artificial chromosome. The vector may contain any means for assuring self-replication. Alternatively, the vector may be one which, when introduced into the host cell, is integrated into the genome and replicated together with the chromosome(s) into which it has been integrated. Furthermore, a single vector or plasmid or two or more vectors or plasmids which together contain the total DNA to be introduced into the genome of the host cell, or a transposon may be used. The vector may be linear or closed circular plasmids and will preferably contain an element(s) that permits stable integration of the vector into the host cell's genome or autonomous replication of the vector in the cell independent of the genome.

In a preferred embodiment, the recombinant expression vector is capable of replicating in yeast. Examples of sequences which enable the vector to replicate in yeast are the yeast plasmid 2 µm replication genes REP 1-3 and origin of replication.

The vectors of the present invention preferably contain one or more selectable markers which permit easy selection of transformed cells. A selectable marker is a gene the product of which provides for biocide or viral resistance, resistance to heavy metals, prototrophy to auxotrophs, and the like. Examples of bacterial selectable markers are the *dal* genes from *Bacillus subtilis* or *Bacillus licheniformis,* or markers which confer antibiotic resistance such as ampicillin, kanamycin, chloramphenicol or tetracycline resistance. Selectable markers for use in a filamentous fungal host cell include *amdS* (acetamidase), *argB* (ornithine carbamoyltransferase), *pyrG* (orotidine-5'-phosphate decarboxylase) and *trpC* (anthranilate synthase). Suitable markers for yeast host cells are ADE2, HIS3, LEU2, LYS2, MET3, TRP1, and URA3. A preferred selectable marker for yeast is the *Schizosaccharomyces pompe* TPI gene (Russell (1985) Gene 40:125-130).

In the vector, the polynucleotide sequence is operably connected to a suitable promoter sequence. The promoter may be any nucleic acid sequence which shows transcriptional activity in the host cell of choice including mutant, truncated, and hybrid promoters, and may be obtained from genes encoding extra-cellular or intra-cellular polypeptides either homologous or heterologous to the host cell.

In a yeast host, useful promoters are the *Saccharomyces cerevisiae* MFα1, TPI, ADHm Gal or PGK promoters.

The polynucleotide construct of the invention will also typically be operably connected to a suitable terminator. In yeast a suitable terminator is the TPI terminator (Alber et al. (1982) J. Mol. Appl. Genet. 1:419-434) or the CYC1 terminator.

The procedures used to ligate the polynucleotide sequence of the invention, the promoter and the terminator, respectively, and to insert them into a suitable vector containing the information necessary for replication in the selected host, are well known to persons skilled in the art. It will be understood that the vector may be constructed either by first preparing a DNA construct containing the entire DNA sequence encoding the precursor molecule of the invention, and subsequently inserting this fragment into a suitable expression vector, or by sequentially inserting DNA fragments containing genetic information for the individual elements followed by ligation.

The host cell is a yeast cell. The yeast organism used in the process of the invention may be any suitable yeast organism which, on cultivation, produces large amounts of the precursor molecule. Examples of suitable yeast organisms are strains selected from the yeast species *Saccharomyces cerevisiae, Saccharomyces kluyveri, Schizosaccharomyces pombe, Sacchoromyces uvarum, Kluyveromyces lactis, Hansenula polymorpha, Pichia pastoris, Pichia methanolica, Pichia kluyveri, Yarrowia lipolytica, Candida sp., Candida utilis, Candida cacaoi, Geotrichum sp.*, and *Geotrichum fermentans.*

The transformation of the yeast cells may for instance be effected by protoplast formation followed by transformation in a manner known *per se.* The medium used to cultivate the cells may be any conventional medium suitable for growing yeast organisms. The secreted precursor of the invention may then be recovered from the medium by conventional procedures including separating the yeast cells from the medium by centrifugation, filtration or catching the precursor by an ion exchange matrix or by a reverse phase absorption matrix, precipitating the proteinaceous components of the supernatant or filtrate by means of a salt, e.g. ammonium sulphate, followed by purification by a variety of chromatographic procedures, e.g. ion exchange chromatography, affinity chromatography, or the like.

In the present text, the designation "**an analogue**" is used to designate a peptide wherein one or more amino acid residues of the parent peptide have been substituted by another amino acid residue and/or wherein one or more amino acid residues of the parent peptide have been deleted and/or wherein one or more amino acid residues have been added to the parent peptide. Such addition can take place either at the N-terminal end or at the C-terminal end of the parent peptide or both.

The term "**derivative**" is used in the present text to designate a peptide in which one or more of the amino acid residues of the parent peptide have been chemically modified, *e.g.* by alkylation, acylation, ester formation or amide formation.

The amino acid residues are either indicated by the one letter or the three letter code.

### BRIEF DESCRIPTION OF THE DRAWINGS

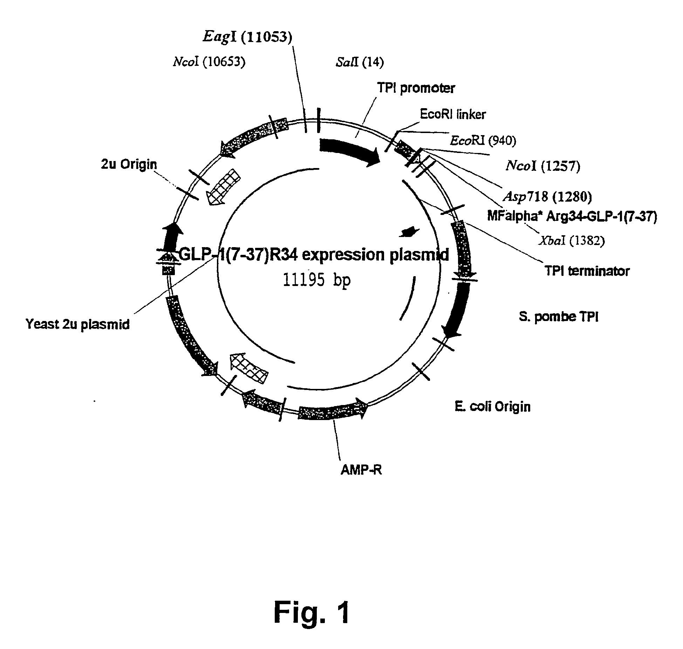

Fig. 1 shows the plasmid pKV304 which contain DNA encoding Arg³⁴GLP-1₍₇₋₃₇₎ under regulatory control of the TPI promoter and -terminator and the MFalpha prepro sequence. This plasmid is the starting plasmid for making expression plasmids for the precursor molecules according to the present invention.

### EXAMPLE 1

### Expression of N-terminallv extended Arg³⁴GLP-1₍₇₋₃₇₎

The host strain ME1719 is a diploid strain and has a phenotype which lacks two aspartyl protease activities, i.e. YPS1 (previously called YAP3) which cleaves C-terminal side of mono- or dibasic amino acid residues (Egel-Mitani, et al., YEAST 6: 127-137, 1990) and PEP4 a vacuolar protease A responsible for activation of other proteases such as protease B, carboxypeptidase Y, aminopeptidase I, RNase, alkaline phosphatase, acid threhalase and exopolyphosphatase. Moreover the triose phosphate isomerase gene (TPI) has been disrupted which phenotype makes it possible to utilize glucose in transformants grown on glucose containing medium. The genetic background of ME1719 is MAT**a**/α Δyps1::ura3/Δyps1::URA3 pep4-3/pep4-3 Δtpi::LEU2/Δtpi::LEU2 leu2/leu2 Δura3/ Δura3.

Expression plasmids containing the N-terminally extended Arg³⁴GLP-1₍₇₋₃₇₎ were made as follows: Plasmid pKV304 containing DNA encoding Arg³⁴GLP-1₍₇₋₃₇₎ without an N-terminal extension was digested with either Eagl+Ncol or Eagl+Asp718. After agarose electrophoresis and GeneClean^{™} III purification, fragments of 1.4 kb and 10 kb, respectively were isolated. Oligonucleotide adaptors corresponding to various N-terminal extensions of Arg³⁴GLP-1₍₇₋₃₇₎ containing Ncol and Asp718 cleavage sites were likewise purified as described above. The 1.4 kb fragment (Eagl+Ncol), 10 kb fragment (Eagl+Asp718) and the adaptor fragment designed for the N-terminal extension of Arg³⁴GLP-1(₇₋₃₇) (Ncol+Asp718) were ligated and transformed in E. coli strain MT172 and plasmid DNA was sequenced to verify the correct N-terminally extended Arg³⁴GLP-1₍₇₋₃₇₎.

Plasmid DNA was then transformed into yeast strain ME1719 and yeast transformants were isolated twice on MUPD selective plates. Yeast cells were cultured in 5 ml MUPD medium for 3 days at 30°C and culture supernatants were analyzed by HPLC and MALDI-MS (Matrix Assisted Laser Desorption/Inonisation Mass Spectrometry). MUPD medium consists of 25 g yeast extract (Bacto), 5 g KH₂PO₄, 1,5 g MgSO₄.7H₂O, ion exchanged water to 1 liter and pH adjusted to 6,0 with 5 N H₂SO₄ before autoclavation at 121°C, 20 min. 30 g glucose was separately autoclaved in a 50%w/v concentration and added aseptically.

Table 1 shows the different GLP-1 precursors and the yield compared to a control with no N-terminal extension.

**Table 1**

| Extension | Polypeptide | Yield % of control |
|---|---|---|
| None (control) | Arg³⁴GLP-1₍₇₋₃₇₎ | 100 |
| EEK | Arg³⁴GLP-1₍₇₋₃₇₎ | 206 |
| EEAEK (SEQ ID NO:45) | Arg³⁴ GLP-1(₇₋₃₇₎ | 217 |
| HK | Arg³⁴GLP-1₍₇₋₃₇₎ | 63 |
| E(EA)HK (SEQ ID NO:1) | Arg³⁴GLP-1₍₇₋₃₇₎ | 233 |
| E(EA)₂ HK (SEQ ID NO:2) | Arg³⁴ GLP-1₍₇₋₃₇₎ | 208 |
| E(EA)₃HK (SEQ ID NO:3) | Arg³⁴GLP-1₍₇₋₃₇₎ | 223 |
| EEGHK (SEQ ID NO:4) | Arg³⁴GLP-1₍₇₋₃₇₎ | 171 |
| EHPK(SEQ ID NO:5) | Arg³⁴GLP-1₍₇₋₃₇₎ | 132 |
| EEGEPK (SEQ ID NO:6) | Arg³⁴GLP-1₍₇₋₃₇₎ | 211 |
| EEHCK (SEQ ID NO:7) | Arg³⁴GLP-1₍₇₋₃₇₎ | 58 |
| EEHHK (SEQ ID NO:8) | Arg³⁴GLP-1₍₇₋₃₇₎ | 125 |
| EHHHK (SEQ ID NO:9) | Arg³⁴GLP-1₍₇₋₃₇₎ | 72 |
| EHAHK (SEQ ID NO:10) | Arg³⁴GLP-1₍₇₋₃₇₎ | 76 |
| EGAHK (SEQ ID NO: 11) | Arg³⁴GLP-1₍₇₋₃₇₎ | 97 |
| EHGHGK (SEQID NO:12) | Arg³⁴GLP-1₍₇₋₃₇₎ | 73 |
| EEAHELK (SEQ ID NO:13) | Arg³⁴GLP-1₍₇₋₃₇₎ | 220 |
| EEAHEIK (SEQ ID NO:14) | Arg³⁴GLP-1₍₇₋₃₇₎ | 128 |
| EEAHEVK (SEQ ID NO:15) | Arg³⁴GLP-1₍₇₋₃₇₎ | 217 |
| EEAHEMK (SEQ ID NO:16) | Arg³⁴GLP-1₍₇₋₃₇₎ | 232 |
| EEAHEFK (SEQ ID NO:17) | Arg³⁴GLP-1₍₇₋₃₇₎ | 226 |
| EEAHEYK (SEQ ID NO:18) | Arg³⁴GLP-1₍₇₋₃₇₎ | 200 |
| EEAHEWK (SEQ ID NO:19) | Arg³⁴GLP-1₍₇₋₃₇₎ | 200 |
| EEGNTTPK (SEQ ID NO:20) | Arg³⁴GLP-1₍₇₋₃₇₎ | 216 |
| EEGNETEPK (SEQ ID NO:21) | Arg³⁴ GLP-1₍₇₋₃₇₎ | 145 |
| EEGNDTEPK (SEQ ID NO:22) | Arg³⁴GLP-1₍₇₋₃₇₎ | 175 |
| EEGNTTEPK (SEQ ID NO:23) | Arg³⁴GLP-1₍₇₋₃₇₎ | 31* |
| QDAHK (SEQ ID NO:24) | Arg³⁴GLP-1₍₇₋₃₇₎ | 55 |
| QDTAK (SEQ ID NO:25) | Arg³⁴GLP-1₍₇₋₃₇₎ | 66 |
| DDDDK (SEQ ID NO:26) | Arg³⁴GLP-1₍₇₋₃₇₎ | 190 |
| EAEAWHWLK (SEQ ID NO:27) | Arg³⁴GLP-1₍₇₋₃₇₎ | 36 |
| EAEAEAWHWLK (SEQ ID NO:28) | Arg³⁴GLP-1₍₇₋₃₇₎ | 34. |
| EEAEAWHWLK (SEQ ID NO:29) | Arg³⁴GLP-1₍₇₋₃₇₎ | 20 |
| EEEAWHWLK (SEQ ID NO:30) | Arg³⁴GLP-1₍₇₋₃₇₎ | n.d. |
| LDGRLEALK (SEQ ID NO:31) | Arg³⁴GLP-1₍₇₋₃₇₎ | 58 |
| EELDGRLEALK (SEQ ID NO:32) | Arg³⁴GLP-1₍₇₋₃₇₎ | 211 |
| EELDARLEALK (SEQ ID NO:33) | Arg³⁴GLP-1₍₇₋₃₇₎ | 239 |

| | | |
|---|---|---|
| * Product mainly hyperglycosylated | | |

### EXAMPLE 2

### Acylation of EEAHK(SEQ ID NO:1)- Arg³⁴GLP-1₍₇₋₃₇₎ in the presence and absence of divalent or trivalent metal ions

The GLP-1 analogue was produced as described in Example 1. The fermentation broth was clarified by centrifugation and 2620 ml of supernatant was diluted to 7900 ml and pH was adjusted to pH 3.1. The final conductivity was 4.9 mS/cm. A 2.6 x 100 cm column packed with 100 ml Pharmacia Streamline^{®} SP Code no. 17-0993-05 was equilibrated and fluidised as recommended by the supplier (Pharmacia booklet 18-1124-26, Expanded Gel Adsorption, Principles and Methods) employing a 0.025M citrate buffer pH 3.1 and subsequently eluted by 0,5M Tris base at a flow of 0.5 ml/min. The fractions containing the GLP1 analogue was identified by analytical RP-HPLC employing a gradient of CH₃CN in 0.010M Tris, 0.015M Na₂SO₄ pH 7.4 with diluted H₂SO₄. The volume of the pooled samples was 100 ml containing 361 mg of the GLP-1 analogue and the purity was 72.4%. The sample was further purified by preparative RP-HPLC. The buffer system consisted of an A-buffer 0.010M Tris, 0.015M Na₂SO₄ and 20% ethanol v/v pH 7.5 with diluted H₂SO₄ and an B-buffer consisting of 70% ethanol. Aliquots corresponding to 90 mg GLP1 analogue was applied to a HPLC column (250 x 20)mm packed with Nucleosil 300A, 7µm, C4 obtained from Macherey-Nagel, D, equilibrated with 10%B the sample was eluted with a linear gradient from 10%B to 90%B in a total of 720 ml at a flow rate of 6 ml min. The eluent was monitored at 214 nm and 276 nm. The samples containing the GLP1 analogue were pooled, diluted with one volume of water, adjusted to pH 5.0 and cooled to 4°C. The precipitate was isolated by centrifugation and lyophilised. 286 mg was obtained and the final purity was 98.0%.

Acylation of the GLP-1 analogue was performed employing 10 mg samples of purified analogue. The sample was dissolved in 0.5 ml 0.05M Na₂CO₃ and incubated at 15°C. Glu(ONSU)N-hexadecanoyl methylester was dissolved in 0.5 ml CH₃CN and added to the analogue solution. Samples were taken before addition of reagent and after 15 and 30 minutes and after termination of the reaction with quenching buffer. The samples were added quenching buffer and diluted with 20% vol/vol ethanol and analysed by analytical RP-HPLC. Acylation in the presence of divalent metal ions was conducted by addition of the appropriate volume of a 0.1M solution of the metal ion before addition of the amino acid acylation reagent. The volume of Na₂CO₃ buffer was adjusted accordingly. Zn²⁺ acetate was used in experiment with Zn²⁺ as the metal ion.

### Optimization of acylation of EEAHK(SEQ ID NO:1)- Arg³⁴GLP-1₍₇₋₃₇₎-Lys²⁶ γ-Glu-hexadecanoyl in the presence of Zn²⁺

Addition of more than 2 equivalents of Zn²⁺ was associated by precipitation of the sample. However, the yield improved when 2 equivalents of Zn²⁺ was used for acylation together with a surplus of acylation reagent. Thus, a yield of 52% was obtained together with 7% of desamidated acylation product, which is superior to 42.4% yield and 5.1% desamidated product obtained in the absence of Zn²⁺.

The results are shown in Table 2 and Table 3 below.

**Table 2**

| **Acylation of EEAHK(SEQ ID NO:1)-Arg³⁴GLP-1₍₇₋₃₇) at pH 10.2, 50% CH₃CN by 1.3 equivalents Glu(ONSU)N-hexadecanoyl methylester** | | | | | | | |
|---|---|---|---|---|---|---|---|
| Reaction | 0 min | 15 min | | 30 min | | 30 min stop | |
| GLP-1 | | GLP-1 product | | GLP-1 product | | GLP-1 product | |
| 0.0 equivalents Zn²⁺ | 98.% | 36.2% | 41.4% | 35.5% | 42.4%* | 36.1% | 45.2 |
| 0.5 equivalents Zn²⁺ | 98.3% | 30.2% | 42.9% | | | 32.6% | 41.7% |
| 1.0 equivalents Zn²⁺ | 99.5% | 33.8% | 38.6% | | | 34.8% | 34.6% |
| 1.5 equivalents Zn²⁺ | 99.3% | 30.7% | 36.3% | 29.2% | 35.5% | 31.7% | 34,2% |
| 2.0 equivalents Zn²⁺ | 99.2% | 28.1% | 44.3% | 22.9% | 46.8% | 30.0% | 42.8% |
| 2.5 equivalents Zn²⁺ | 99,3% | 25.0% | 39.8% | 21.3% | 46.6% | 22.5% | 45.0% |

| | | | | | | | |
|---|---|---|---|---|---|---|---|
| *% 5.1% of desamidated product was obtained in this synthesis | | | | | | | |

**Table 3**

| **Acylation of EEAHK(SEQ ID NO:1)-Arg³⁴GLP-1₍₇₋₃₇₎ at pH 10.2, 50% CH₃CN by 2 and 3 equivalents Glu(ONSU)N-hexadecanoyl methylester and 2 equivalents Zn²⁺** | | | | | | | |
|---|---|---|---|---|---|---|---|
| Reaction Acylation reagent | 0 min | 15 min | | 30 min | | 30 min stop | |
| | | GLP-1 product | | GLP-1 product | | GLP-1 product | |
| 2 equivalents acylation reagent | 98.5% | 16.9% | 37.8% | 11.6% | 39.0% | 12.9% | 38.4% |
| 3 equivalents acylation reagent | 98.3% | 6.0% | 35.7% | 3.8% | 33.2% | | |

| Reaction Acylation reagent | 0 min | 15 min | | 30 min | | 30 min stop | |
|---|---|---|---|---|---|---|---|
| | | GLP-1 product | | GLP-1 product | | GLP-1 product | |
| 2 equivalents Zn²⁺ | 98.1 | 27.8% | 52.0% | 16.4% | 52.0%* | 19.9% | 46,1% |
| 2 equivalents acylation reagent | | | | | | | |
| 2 equivalents Zn²⁺ | 98.6% | 13.2% | 36.6% | 15.8% | 38.4% | 13.2% | 41.6% |
| 3 equivalents acylation reagent | | | | | | | |

| | | | | | | | |
|---|---|---|---|---|---|---|---|
| *% 7.0% of desamidated product was obtained in this synthesis. | | | | | | | |

### Synthesis of Arg³⁴GLP-1₍₇₋₃₇₎-Lys²⁶ γ-Glu-hexadecanoyl in the presence of Zn²⁺

EEAHK(SEQ ID NO:1)-Arg³⁴GLP-1₍₇₋₃₇₎ was acylated by addition of 2 equivalents of acylation reagent in the presence of 2 equivalents Zn²⁺ for 15 min. as described above. A total of 15µl was applied to an analytical RP-HPLC column using a TFA/CH₃CN/H₂O buffer system. The purified product corresponded to 57 µg represent a synthesis yield of 46%. The product was dried and subsequently dissolved in 0.025ml 0.1m NaOH at 0°C and incubated for 20 min. After hydrolysis of the methylester, the sample was then added 15 µl 0.1m HCl and 5 µl 0.1m Tris.HCl and a pH indicator paper measured a pH of approximately pH 8-9. The sample was then added 10µl CH₃CN and thereafter 1µl 1mg/ml *Achromobacter lyticus* protease I (EC 3.4.21.50) and incubated for 30 min at room temperature. A total of 10 µl was applied to the RP-HPLC system as described above and the most prominent of the 3 peaks corresponding 66.2% was further characterised and found to be identical to the desired product. The results are shown in Table 4 below.

**Table 4**

| Peak | MW calculated | MW found | Identity | Yield |
|---|---|---|---|---|
| Peak 1 | - | No signal | GLP-1 analogue | 8.5% |
| Peak 2 | 3749.29 | 3748.41 | Arg³⁴ GLP-1₍₇₋₃₇₎-Lys²⁶- γ-Glu-hexadecanoyl | 66.2% |
| Peak 3 | 3749.29 | 3761.8 | Unknown derivative of GLP-1 | 19.6% |

### Example 3

### Acylation and ALP-processing of EEAEK(SEQ ID NO: 45)-Arg³⁴GLP-1₍₇₋₃₇₎

The GLP-1 precursor EEAEK(SEQ ID NO:43)-Arg³⁴GLP-1₍₇₋₃₇₎ was expressed in yeast as described above and recovered by adjusting the ionic strength to 7 in the fermentation supernatant and binding to a cationic column SP Sepharose Fast Flow XL (Amersham-Pharmacia). The precursor was eluted with a gradient 0-100% of 50 mM formic acid + 1 M NaCl in 50 mM formic acid. After 10 column volumes the column was washed with 2 volumes of 50 mM formic acid followed by elution with 0.5 M glycine pH 9 for 3.5 column volumes. The fractions were analysed by MALDI-MS and the precursor pools were identified. These pools were applied on Reverse Phase HPLC a ZORBAX 300SB-CN column (9,4x250 mm). Elution was carried out with a gradient of 30-70 % of buffer B (0,1 % TFA/ 80% ethanol) in buffer A (0,1 % TFA). Fractions were identified by UV-pattern and MALDI-MS. Pools were collected, the ethanol evaporated in vacuum and the product was lyophilised.

5 mg of lyophilised precursor EEAEK(SEQ ID NO:45)-Arg³⁴GLP-1₍₇₋₃₇₎ was dissolved in 350 µl water and 700 µl NMP (N-methyl-pyrrolidon-2) at room temperature and 4 equivalents of N-hexadecanoyl-/-glutamic acid-α-tert-butyl ester-γ-succinylimidyl ester was added at time zero. The pH was adjusted to 9.5 with EDIPA (ethyl di-isopropyl amin) and kept constant at 9.5 by addition of EDIPA. After 60 minutes the reaction was stopped by addition of 208 µl of a solution of 1% glycin in water. Analysis of the reaction mixture showed that 24% was unreacted product. 61% was monoacylated in position Lys²⁶. 8% was diacylated in Lys²⁶ and at the N-terminal amino group. 7% was diacylated in Lys⁶ and Lys²⁶. The mono- and diacylated molecules were converted to monoacylated Arg³⁴GLP-1₍₁₋₃₇₎ (69%) by processing at the lysine clevage site with ALP according to well established procedures. The result was confirmed by HPLC and MALDI-MS.

### Example 4

### Acylation and cleavage of EELDARLEALK(SEQ ID NO:33)-Arg³⁴GLP-1₍₁₋₃₇₎, EEAHEYK(SEQ ID NO:18)-Arg³⁴GLP-1₍₁₋₃₇₎ and DDDDK(SEQ ID NO: 26)-Arg³⁴GLP-1₍₁₋₃₇₎

The GLP-1 precursors EELDARLEALK(SEQ ID NO:33)-Arg³⁴GLP-1₍₁₋₃₇₎, EEAHEYK(SEQ ID NO:18)-Arg³⁴GLP-1₍₁₋₃₇₎ and DDDDK(SEQ ID NO: 26)-Arg³⁴GLP-1₍₁₋₃₇₎ were all expressed in yeast and purified as described in Example 3.
Acylation was carried out as described in Example3. 1.3 Equivalents were used of hexadecanoyl-/-glutamic acid-α-tert-butyl ester-γ-succinylimidyl ester. HPLC and MALDI-MS analysis revealed in all cases ∼ 15-30% unreacted substrate and around 20 % diacylated product (Lys⁶, Lys²⁶) after hydrolysis with ALP directly in the acylation mixture. The results are shown in table 5.

**Table 5**

| **Only monoacylated N-terminal extended Lys²⁶-GLP1 and diacylated N-terminal extended Lys⁶, Lys²⁶-GLP1 peaks determined** | | |
|---|---|---|
| Extension | % Monoacylated Lys²⁶ | % Diacylated Lys⁶, Lys^{26**} |
| DDDDK (SEQ ID NO:26) | 80 | 20 |
| EELDARLEALK (SEQ ID NO:33) | 76 | 24 |
| EEAHEYK (SEQ ID NO:18) | 76 | 24 |

| | | |
|---|---|---|
| ** N-terminal extended (not cleaved) | | |

## Claims

1. A method for making a polypeptide comprising at least one lysine residue being acylated in its ε-amino group, said method comprising the following steps:
(i) culturing a yeast cell comprising a polynucleotide sequence encoding a precursor molecule of said polypeptide under suitable conditions for expression of said precursor molecule, the precursor molecule comprising the desired polypeptide and an N-terminal extension said N-terminal extension being cleavable from the desired polypeptide at a lysine cleavage site and having the following formula:
Xₙ------X₁ - Lys
wherein Lys is a cleavage site and Xₙ------X₁ is a peptide sequence of from 2-14 amino acid residues in length having the function of preventing or minimizing acylation of the free ε-amino group in the Lys cleavage site in step (iii) and having the further function of protecting the expressed precursor polypeptide from endoproteolytic cleavage with the proviso that no X is Lys and that at least one X is His or Glu or Asp;
(ii) separating the expressed precursor molecule from the culture broth;
(iii) preferentially acylating the ε-amino group of at least one lysine residue in the desired polypeptide without acylating the ε-amino group of the lysine cleavage site in the N-terminal extension;
(iv) removing the N-terminal extension from the acylated precursor molecule by enzymatic cleavage and
(v) isolating the acylated polypeptide by suitable means.

2. A method according to claim 1, wherein the polypeptide is monoacylated.

3. A method according to claim 1, wherein the Glu or Asp residues are positioned between 1 to 5 residues from the lysine cleavage site.

4. A method according to claim 1, wherein the N-terminal extension comprises a Glu-Glu-sequence.

5. A method according to claim 1, wherein one or more histidine residues in the N-terminal extension are positioned 1 - 4 residues from the lysine cleavage site.

6. A method according to claim 1, wherein Xₙ------X₁ is of 2-12 amino acid residues in length.

7. A method according to claim 1, wherein the first and second X from the N-terminal end of the N-terminal extension are selected from the group consisting of Glu and Asp.

8. A method according to claim 1, wherein Xₙ ----- X₁ - Lys is selected from the group consisting of Glu-Glu-Ala-His-Lys(SEQ ID NO:1); Glu-(Glu-Ala)₂-His-Lys(SEQ ID NO:2); Glu-(Glu-Ala)₃His-Lys(SEQ ID NO:3); Glu-Glu-Gly-His-Lys( SEQ ID NO:4); Glu-His-Pro-Lys(SEQ ID NO:5); Glu-Glu-Gly-Glu-Pro-Lys(SEQ ID NO:6); Glu-Glu-His-Cys-Lys(SEQ ID NO:7); Glu-Glu-His-His-Lys(SEQ ID NO:8); Glu-His-His-His-Lys(SEQ ID NO:9); Glu-His-Ala-His-Lys(SEQ ID NO:10); Glu-Gly-Ala-His-Lys(SEQ ID NO:11); Glu-His-Gly-His-Gly-Lys(SEQ ID NO:12); Glu-Glu-Ala-His-Glu-Leu-Lys(SEQ ID NO:13); Glu-Glu-Ala-His-Glu-Ile-Lys(SEQ ID NO:14); Glu-Glu-Ala-His-Glu-Val-Lys(SEQ ID NO:15); Glu-Glu-Ala-His-Glu-Met-Lys(SEQ ID NO:16); Glu-Glu-Ala-His-Glu-Phe-Lys(SEQ ID NO:17); Glu-Glu-Ala-His-Glu-Tyr-Lys(SEQ ID NO:18); Glu-Glu-Ala-His-Glu-Trp-Lys(SEQ ID NO:19); Glu-Glu-Gly-Asn-Thr-Thr-Pro-Lys(SEQ ID NO:20); Glu-Glu-Gly-Asn-Glu-Thr-Glu-Pro-Lys(SEQ ID NO:21), Glu-Glu-Gly-Asn-Asp-Thr-Glu-Pro-Lys(SEQ ID NO:22); Glu-Glu-Gly-Asn-Thr-Thr-Glu-Pro-Lys(SEQ ID NO: 23); Gln-Asp-Ala-His-Lys(SEQ ID NO:24); Glu-Glu-Lys; Asp-Asp-Asp-Asp-Lys(SEQ ID NO:26); Glu-Glu-Ala-Glu-Ala-Trp-His-Trp-Leu-Lys(SEQ ID NO:29); Glu-Glu-Glu-Ala-Trp-His-Trp-Leu-Lys(SEQ ID NO:30); Leu-Asp-Gly-Arg-Leu-Glu-Ala-Leu-Lys(SEQ ID NO:31); Glu-Glu-Leu-Asp-Gly-Arg-Leu-Glu-Ala-Leu-Lys (SEQ ID NO:32); Glu-Glu-Leu-Asp-Ala-Arg-Leu-Glu-Ala-Leu-Lys(SEQ ID NO:33); Asp-Thr-His-Lys(SEQ ID NO:34); Asp-Ala-His-Lys(SEQ ID NO:35); Glu-His-His-Gly-His-Gly-Lys(SEQ ID NO:36); Asp-Ser-His-Lys(SEQ ID NO:37); Gln-Asp-Thr-His-Lys(SEQ ID NO:38); Glu-Ala-Glu-Ala-Glu-Ala-Gln-Asp-Thr-His-Lys(SEQ ID NO:39); Glu-Ala-Glu-Ala-Gln-Asp-Thr-His-Lys(SEQ ID NO:40); Glu-Ala-Gln-Asp-Thr-His-Lys(SEQ ID NO:41); Trp-His-Trp-Leu-Lys(SEQ ID NO:42); Glu-Glu-Trp-His-Trp-Leu-Lys(SEQ ID NO:43); Glu-Glu-Glu-Ala-Trp-His-Trp-Leu-Lys(SEQ ID NO:44); Glu-Glu-Ala-Glu-Lys(SEQ ID NO:45); Glu-Glu-Gly-Glu-Pro-Lys(SEQ ID NO:46); Glu-Ala-Gln-Asp-Ala-His-Lys(SEQ ID NO:47); Glu-Ala-Glu-Ala-Gln-Asp-Ala-His-Lys(SEQ ID NO:48); and Glu-Ala-Glu-Ala-Glu-Ala-Gln-Asp-Ala-His-Lys(SEQ ID NO:49);

9. A method according to claim 1, wherein Xₙ ----- X₁- Lys is selected from the group consisting of Glu-Glu-Ala-His-Lys(SEQ ID NO:1); Glu-(Glu-Ala)₂-His-Lys(SEQ ID NO:2); Glu-(Glu-Ala)₃His-Lys(SEQ ID NO:3); Glu-Glu-Gly-His-Lys(SEQ ID NO:4); Glu-His-Pro-Lys(SEQ ID NO:5);.Glu-Glu-Gly-Glu-Pro-Lys(SEQ ID NO:6); Glu-Glu-His-Cys-Lys(SEQ ID NO:7); Glu-Glu-His-His-Lys(SEQ ID NO:8); Glu-His-His-His-Lys(SEQ ID NO:9); Glu-His-Ala-His-Lys(SEQ ID NO:10); Glu-Gly-Ala-His-Lys(SEQ ID NO:11); Glu-His-Gly-His-Gly-Lys(SEQ ID NO:12); Glu-Glu-Ala-His-Glu-Leu-Lys(SEQ ID NO:13); Glu-Glu-Ala-His-Glu-Ile-Lys(SEQ ID NO:14); Glu-Glu-Ala-His-Glu-Val-Lys(SEQ ID NO:15); Glu-Glu-Ala-His-Glu-Met-Lys(SEQ ID NO:16); Glu-Glu-Ala-His-Glu-Phe-Lys(SEQ ID NO:17); Glu-Glu-Ala-His-Glu-Tyr-Lys(SEQ ID NO:18); Glu-Glu-Ala-His-Glu-Trp-Lys(SEQ ID NO:19); Gln-Asp-Ala-His-Lys(SEQ ID NO:24); Glu-Glu-Glu-Ala-Trp-His-Trp-Leu-Lys(SEQ ID NO:30); Asp-Thr-His-Lys(SEQ ID NO:34); Glu-His-His-Gly-His-Gly-Lys(SEQ ID NO:36); Asp-Ser-His-Lys(SEQ ID NO:37); Gln-Asp-Thr-His-Lys(SEQ ID NO:38); Glu-Ala-Glu-Ala-Glu-Ala-Gln-Asp-Thr-His-Lys(SEQ ID NO:39); Glu-Ala-Glu-Ala-Gln-Asp-Thr-His-Lys( SEQ ID NO:40); Glu-Ala-Gln-Asp-Thr-His-Lys(SEQ ID NO:41); Trp-His-Trp-Leu-Lys(SEQ ID NO:42); Glu-Glu-Trp-His-Trp-Leu-Lys(SEQ ID NO:43); Glu-Glu-Glu-Ala-Glu-Ala-Trp-His-Trp-Leu-Lys(SEQ ID NO:44); Glu-Ala-Gln-Asp-Ala-His-Lys(SEQ ID NO:47); Glu-Ala-Glu-Ala-Gln-Asp-Ala-His-Lys(SEQ ID NO:48); and Glu-Ala-Glu-Ala-Glu-Ala-Gln-Asp-Ala-His-Lys(SEQ ID NO:49).

10. A method according to claim 1, wherein Xₙ ----- X₁ - Lys is Glu-Glu-Ala-Glu-Lys, SEQ ID NO:45; Glu-Glu-Gly-Glu-Pro-Lys, SEQ ID NO:46 or Glu-Glu-Lys.

11. A method according to claim 1, wherein the Xₙ ----- X₁ - Lys is selected from group consisting of Glu-Glu-Ala-Glu-Ala-Trp-His-Trp-Leu-Lys(SEQ ID NO:29), Glu-Glu-Glu-Ala-Trp-His-Trp-Leu-Lys(SEQ ID NO:30); Leu-Asp-Gly-Arg-Leu-Glu-Ala-Leu-Lys(SEQ ID NO:31); Glu-Glu-Leu-Asp-Gly-Arg-Leu-Glu-Ala-Leu-Lys(SEQ ID NO:32); Glu-Glu-Leu-Asp-Ala-Arg-Leu-Glu-Ala-Leu-Lys(SEQ ID NO:33); Glu-Glu-Trp-His-Trp-Leu-Lys(SEQ ID NO:43); and Glu-Glu-Glu-Ala-Glu-Ala-Trp-His-Trp-Leu-Lys(SEQ ID NO:44).

12. A method according to claim 1, wherein the N-terminal extension is selected from the group consisting of Glu-Glu-Gly-Asn-Thr-Thr-Pro-Lys(SEQ ID NO:20), Glu-Glu-Gly-Asn-Glu-Thr-Glu-Pro-Lys(SEQ ID NO:21), Glu-Glu-Gly-Asn-Asp-Thr-Glu-Pro-Lys(SEQ ID NO:22) and Glu-Glu-Gly-Asn-Thr-Thr-Glu-Pro-Lys (SEQ ID NO: 23).

13. A method according to claim 1, wherein the enzymatic cleavage in step (iv) is conducted by use of a lysine endopeptidase such as the *Achromobacter lyticus* protease I.

14. A method according to claim 1, wherein the desired polypeptide belongs to the GRF (growth hormone releasing factor) family of peptides having a His or Tyr in the N-terminal position and Ser, Ala or Gly in the next position

15. A method according to claim 14, wherein the desired polypeptide is GLP-1 or GLP-2 or a GLP-1 or GLP2 analogue or derivative.

16. A method according to claim 15, wherein the GLP-1 analogue is Arg³⁴GLP1₍₇₋₃₇₎ acylated in position Lys²⁶.

17. A method according to claim 1, wherein the acylation step (iii) is performed in an organic solvent or in a mixture of water and an organic solvent in the presence of metal ions.

18. A method according to claim 1, wherein the acylation step is conducted at a pH between 7 and 12.

## Patentansprüche

1. Verfahren zur Herstellung eines Polypeptids, das mindestens einen in seiner ε-Aminogruppe acylierten Lysinrest umfasst, wobei das Verfahren die folgenden Schritte umfasst:
(i) Kultivieren einer Hefezelle, die eine ein Vorläufermolekül des Polypeptids kodierende Polynukleotidsequenz umfasst, unter zur Expression des Vorläufermoleküls geeigneten Bedingungen, wobei das Vorläufermolekül das gewünschte Polypeptid und eine N-terminale Verlängerung umfasst, wobei die N-terminale Verlängerung an einer Lysinspaltstelle von dem gewünschten Polypeptid abspaltbar ist und die folgende Formel aufweist:
Xₙ------X₁ - Lys
wobei Lys für eine Spaltstelle steht und Xₙ-----X₁ für eine Peptidsequenz mit einer Länge von 2 - 14 Aminosäureresten mit der Funktion des Verhinderns oder Minimierens einer Acylierung der freien ε-Aminogruppe in der Lys-Spaltstelle in Schritt (iii) und mit der weiteren Funktion des Schützens des exprimierten Vorläuferpolypeptids vor einer endoproteolytischen Spaltung steht, mit der Maßgabe, dass kein X für Lys steht und dass mindestens ein X für His oder Glu oder Asp steht;
(ii) Abtrennen des exprimierten Vorläufermoleküls von der Kulturbrühe;
(iii) vorzugsweise Acylieren der ε-Aminogruppe an mindestens einem Lysindrest im gewünschten Polypeptid ohne Acylieren der ε-Aminogruppe der Lysinspaltstelle in der N-terminalen Verlängerung;
(iv) Entfernen der N-terminalen Verlängerung von dem Acylierten Vorläufermolekül durch enzymatische Spaltung und
(v) Isolieren des acylierten Polypeptids durch geeignete Mittel.

2. Verfahren nach Anspruch 1, wobei das Polypeptid monoacyliert ist.

3. Verfahren nach Anspruch 1, wobei die Glu- oder Asp-Reste zwischen 1 bis 5 Resten von der Lysinspaltstelle positioniert sind.

4. Verfahren nach Anspruch 1, wobei die N-terminale Verlängerung eine Glu-Glu-Sequenz umfasst.

5. Verfahren nach Anspruch 1, wobei ein oder mehr Histidinrest(e) in der N-terminalen Verlängerung 1-4 Reste von der Lysinspaltstelle positioniert ist (sind).

6. Verfahren nach Anspruch 1, wobei Xₙ-----X₁ eine Länge von 2-12 Aminosäureresten aufweist.

7. Verfahren nach Anspruch 1, wobei das erste und zweite X von dem N-terminalen Ende der N-terminalen Verlängerung ausgewählt sind aus der Gruppe, bestehend aus Glu und Asp.

8. Verfahren nach Anspruch 1, wobei Xₙ ----- X₁ - Lys ausgewählt ist aus der Gruppe, bestehend aus Glu-Glu-Ala-His-Lys(SEQ ID NR:1); Glu-(Glu-Ala)₂-His-Lys(SEQ ID NR:2); Glu-(Glu-Ala)₃His-Lys(SEQ ID NR:3); Glu-Glu-Gly-His-Lys( SEQ ID NR:4); Glu-His-Pro-Lys(SEQ ID NR:5); Glu-Glu-Gly-Glu-Pro-Lys(SEQ ID NR:6); Glu-Glu-His-Cys-Lys(SEQ ID NR:7); Glu-Glu-His-His-Lys(SEQ ID NR:8); Glu-His-His-His-Lys(SEQ ID NR:9); Glu-His-Ala-His-Lys(SEQ ID NR:10); Glu-Gly-Ala-His-Lys(SEQ ID NR:11); Glu-His-Gly-His-Gly-Lys(SEQ ID NR:12); Glu-Glu-Ala-His-Glu-Leu-Lys(SEQ ID NR:13); Glu-Glu-Ala-His-Glu-Ile-Lys(SEQ ID NR:14); Glu-Glu-Ala-His-Glu-Val-Lys(SEQ ID NR:15); Glu-Glu-Ala-His-Glu-Met-Lys(SEQ ID NR:16); Glu-Glu-Ala-His-Glu-Phe-Lys(SEQ ID NR:17); Glu-Glu-Ala-His-Glu-Tyr-Lys(SEQ ID NR:18); Glu-Glu-Ala-His-Glu-Trp-Lys(SEQ ID NR:19); Glu-Glu-Gly-Asn-Thr-Thr-Pro-Lys(SEQ ID NR:20); Glu-Glu-Gly-Asn-Glu-Thr-Glu-Pro-Lys(SEQ ID NR:21), Glu-Glu-Gly-Asn-Asp-Thr-Glu-Pro-Lys(SEQ ID NR:22); Glu-Glu-Gly-Asn-Thr-Thr-Glu-Pro-Lys(SEQ ID NR: 23); Gln-Asp-Ala-His-Lys(SEQ ID NR:24); Glu-Glu-Lys; Asp-Asp-Asp-Asp-Lys(SEQ ID NR:26); Glu-Glu-Ala-Glu-Ala-Trp-His-Trp-Leu-Lys(SEQ ID NR:29); Glu-Glu-Glu-Ala-Trp-His-Trp-Leu-Lys(SEQ ID NR:30); Leu-Asp-Gly-Arg-Leu-Glu-Ala-Leu-Lys(SEQ ID NR:31); Glu-Glu-Leu-Asp-Gly-Arg-Leu-Glu-Ala-Leu-Lys (SEQ ID NR:32); Glu-Glu-Leu-Asp-Ala-Arg-Leu-Glu-Ala-Leu-Lys(SEQ ID NR:33); Asp-Thr-His-Lys(SEQ ID NR:34); Asp-Ala-His-Lys(SEQ ID NR:35); Glu-His-His-Gly-His-Gly-Lys(SEQ ID NR:36); Asp-Ser-His-Lys(SEQ ID NR:37); Gln-Asp-Thr-His-Lys(SEQ ID NR:38); Glu-Ala-Glu-Ala-Glu-Ala-Gln-Asp-Thr-His-Lys(SEQ ID NR:39); Glu-Ala-Glu-Ala-Gln-Asp-Thr-His-Lys(SEQ ID NR:40); Glu-Ala-Gln-Asp-Thr-His-Lys(SEQ ID NR:41); Trp-His-Trp-Leu-Lys(SEQ ID NR:42); Glu-Glu-Trp-His-Trp-Leu-Lys(SEQ ID NR:43); Glu-Glu-Glu-Ala-Trp-His-Trp-Leu-Lys(SEQ ID NR:44); Glu-Glu-Ala-Glu-Lys(SEQ ID NR:45); Glu-Glu-Gly-Glu-Pro-Lys(SEQ ID NR:46); Glu-Ala-Gln-Asp-Ala-His-Lys(SEQ ID NR:47); Glu-Ala-Glu-Ala-Gln-Asp-Ala-His-Lys(SEQ ID NR:48); und Glu-Ala-Glu-Ala-Glu-Ala-Gln-Asp-Ala-His-Lys(SEQ ID NR:49).

9. Verfahren nach Anspruch 1, wobei Xₙ ----- X₁ - Lys ausgewählt ist aus der Gruppe, bestehend aus Glu-Glu-Ala-His-Lys( SEQ ID NR:1); Glu-(Glu-Ala)₂-His-Lys(SEQ ID NR:2); Glu-(Glu-Ala)₃His-Lys(SEQ ID NR:3); Glu-Glu-Gly-His-Lys(SEQ ID NR:4); Glu-His-Pro-Lys(SEQ ID NR:5); Glu-Glu-Gly-Glu-Pro-Lys(SEQ ID NR:6); Glu-Glu-His-Cys-Lys(SEQ ID NR:7); Glu-Glu-His-His-Lys(SEQ ID NR:8); Glu-His-His-His-Lys(SEQ ID NR:9); Glu-His-Ala-His-Lys(SEQ ID NR:10); Glu-Gly-Ala-His-Lys(SEQ ID NR:11); Glu-His-Gly-His-Gly-Lys(SEQ ID NR:12); Glu-Glu-Ala-His-Glu-Leu-Lys(SEQ ID NR:13); Glu-Glu-Ala-His-Glu-Ile-Lys(SEQ ID NR:14); Glu-Glu-Ala-His-Glu-Val-Lys(SEQ ID NR:15); Glu-Glu-Ala-His-Glu-Met-Lys(SEQ ID NR:16); Glu-Glu-Ala-His-Glu-Phe-Lys(SEQ ID NR:17); Glu-Glu-Ala-His-Glu-Tyr-Lys(SEQ ID NR:18); Glu-Glu-Ala-His-Glu-Trp-Lys(SEQ ID NR:19); Gln-Asp-Ala-His-Lys(SEQ ID NR:24); Glu-Glu-Glu-Ala-Trp-His-Trp-Leu-Lys(SEQ ID NR:30); Asp-Thr-His-Lys(SEQ ID NR:34); Glu-His-His-Gly-His-Gly-Lys(SEQ ID NR:36); Asp-Ser-His-Lys(SEQ ID NR:37); Gln-Asp-Thr-His-Lys(SEQ ID NR:38); Glu-Ala-Glu-Ala-Glu-Ala-Gln-Asp-Thr-His-Lys(SEQ ID NR:39); Glu-Ala-Glu-Ala-Gln-Asp-Thr-His-Lys( SEQ ID NR:40); Glu-Ala-Gln-Asp-Thr-His-Lys(SEQ ID NR:41); Trp-His-Trp-Leu-Lys(SEQ ID NR:42); Glu-Glu-Trp-His-Trp-Leu-Lys(SEQ ID NR:43); Glu-Glu-Glu-Ala-Glu-Ala-Trp-His-Trp-Leu-Lys(SEQ ID NR:44); Glu-Ala-Gln-Asp-Ala-His-Lys(SEQ ID NR:47); Glu-Ala-Glu-Ala-Gln-Asp-Ala-His-Lys(SEQ ID NR:48); und Glu-Ala-Glu-Ala-Glu-Ala-Gln-Asp-Ala-His-Lys(SEQ ID NR:49).

10. Verfahren nach Anspruch 1, wobei Xₙ ----- X₁ - Lys für Glu-Glu-Ala-Glu-Lys, SEQ ID NR:45; Glu-Glu-Gly-Glu-Pro-Lys, SEQ ID NR:46 oder Glu-Glu-Lys steht.

11. Verfahren nach Anspruch 1, wobei Xₙ ----- X₁ - Lys ausgewählt ist aus der Gruppe, bestehend aus Glu-Glu-Ala-Glu-Ala-Trp-His-Trp-Leu-Lys(SEQ ID NR:29), Glu-Glu-Glu-Ala-Trp-His-Trp-Leu-Lys(SEQ ID NR:30); Leu-Asp-Gly-Arg-Leu-Glu-Ala-Leu-Lys(SEQ ID NR:31); Glu-Glu-Leu-Asp-Gly-Arg-Leu-Glu-Ala-Leu-Lys(SEQ ID NR:32); Glu-Glu-Leu-Asp-Ala-Arg-Leu-Glu-Ala-Leu-Lys(SEQ ID NR:33); Glu-Glu-Trp-His-Trp-Leu-Lys(SEQ ID NR:43); und Glu-Glu-Glu-Ala-Glu-Ala-Trp-His-Trp-Leu-Lys(SEQ ID NR:44).

12. Verfahren nach Anspruch 1, wobei die N-terminale Verlänferung ausgewählt ist aus der Gruppe, bestehend aus Glu-Glu-Gly-Asn-Thr-Thr-Pro-Lys(SEQ ID NR:20), Glu-Glu-Gly-Asn-Glu-Thr-Glu-Pro-Lys(SEQ ID NR:21), Glu-Glu-Gly-Asn-Asp-Thr-Glu-Pro-Lys(SEQ ID NR:22) und Glu-Glu-Gly-Asn-Thr-Thr-Glu-Pro-Lys (SEQ ID NR: 23).

13. Verfahren nach Anspruch 1, wobei die enzymatische Spaltung in Schritt (iv) durch die Verwendung einer Lysinendopeptidase wie die Protease I von *Achromobacter lyticus* durchgeführt wird.

14. Verfahren nach Anspruch 1, wobei das gewünschte Polypeptid zur Familie von GRF (Wachstumshormon freisetzendem Faktor) von Peptiden mit einem His oder Tyr in der N-terminalen Position und Ser, Ala oder Gly in der nächsten Position gehört.

15. Verfahren nach Anspruch 14, wobei es sich bei dem gewünschten Polypeptid um GLP-1 oder GLP-2 oder ein GLP-1- oder GLP2-Analogon oder -Derivat handelt.

16. Verfahren nach Anspruch 15, wobei es sich bei dem GLP-1-Analogon um Arg³⁴GLP1₍₇₋₃₇₎ handelt, das in der Position Lys²⁶ acyliert ist.

17. Verfahren nach Anspruch 1, wobei der Acylierungsschritt (iii) in einem organischen Lösungsmittel oder in einem Gemisch aus Wasser und einem organischen Lösungsmittel in Gegenwart von Metallionen durchgeführt wird.

18. Verfahren nach Anspruch 1, wobei der Acylierungsschritt bei einem pH-Wert zwischen 7 und 12 durchgeführt wird.

## Revendications

1. Procédé de préparation d'un polypeptide comprenant au moins un résidu de lysine acylé au niveau de son groupe ε-amino, le procédé comprenant les étapes consistant :
(i) à cultiver une cellule de levure comprenant une séquence polynucléotidique codant pour une molécule précurseur dudit peptide dans des conditions convenant à l'expression de ladite molécule précurseur, la molécule précurseur comprenant le polypeptide souhaité et une extension N-terminale, ladite extension N-terminale pouvant être séparée par clivage du polypeptide souhaité au niveau d'un site de clivage de lysine, et ayant la formule suivante :
Xₙ-----X₁ - Lys
où Lys est un site de clivage et Xₙ-----X₁ est une séquence peptidique ayant une taille d'environ 2 à 14 résidus d'acides aminés, ayant pour rôle de prévenir ou de minimiser l'acylation du groupe ε-amino libre dans le site de clivage de Lys de l'étape (iii), et ayant pour rôle supplémentaire de protéger d'un clivage endoprotéolytique le polypeptide précurseur exprimé, à la condition qu'aucun X ne soit Lys, et qu'au moins un X soit His ou Glu ou Asp ;
(ii) à séparer du bouillon de culture la molécule précurseur exprimée ;
(iii) à soumettre à une acylation préférentielle le groupe ε-amino d'au moins un résidu de lysine du polypeptide souhaité, sans acyler le groupe ε-amino du site de clivage de la lysine dans l'extension N-terminale ;
(iv) à éliminer l'extension N-terminale de la molécule précurseur acylée par un clivage enzymatique, et
(v) à isoler par des moyens appropriés le polypeptide acylé.

2. Procédé selon la revendication 1, où le polypeptide est monoacylé.

3. Procédé selon la revendication 1, où les résidus Glu ou Asp sont positionnés à une distance de 1 à 5 résidus du site de clivage de la lysine.

4. Procédé selon la revendication 1, où l'extension N-terminale comprend une séquence Glu-Glu.

5. Procédé selon la revendication 1, où un ou plusieurs résidus d'histidine de l'extension N-terminale sont positionnés à une distance de 1 à 4 résidus du site de clivage de la lysine.

6. Procédé selon la revendication 1, où Xₙ-----X₁ a une taille de 2 à 12 résidus d'acides aminés.

7. Procédé selon la revendication 1, où le premier et le deuxième X provenant du site N-terminal de l'extension N-terminale sont choisis dans le groupe consistant en Glu et Asp.

8. Procédé selon la revendication 1, où Xₙ-----X₁ - Lys est choisi dans le groupe consistant en Glu-Glu-Ala-His-Lys (SEQ ID N°1) ; Glu-(Glu-Ala)₂-His-Lys (SEQ ID N°2) ; Glu-(Glu-Ala) ₃His-Lys (SEQ ID N°3) ; Glu-Glu-Gly-His-Lys (SEQ ID N°4) ; Glu-His-Pro-Lys (SEQ ID N°5) ; Glu-Glu-Gly-Glu-Pro-Lys (SEQ ID N°6) ; Glu-Glu-His-Cys-Lys (SEQ ID N°7) ; Glu-Glu-His-Lys (SEQ ID N°8) ; Glu-His-His-His-Lys (SEQ ID N°9) ; Glu-His-Ala-His-Lys (SEQ ID N°10) ; Glu-Gly-Ala-His-Lys (SEQ ID N°11) ; Glu-His-Gly-His-Gly-Lys (SEQ ID N°12) ; Glu-Glu-Ala-His-Glu-Leu-Lys ( SEQ ID N°13) ; Glu-Glu-Ala-His-Glu-Ile-Lys (SEQ ID N°14) ; Glu-Glu-Ala-His-Glu-Val-Lys (SEQ ID N°15) ; Glu-Glu-Ala-His-Glu-Met-Lys (SEQ ID N°16) ; Glu-Glu-Ala-His-Glu-Phe-Lys (SEQ ID N°17) ; Glu-Glu-Ala-His-Glu-Tyr-Lys (SEQ ID N°18) ; Glu-Glu-Ala-His-Glu-Trp-Lys (SEQ ID N°19) ; Glu-Glu-Gly-Asn-Thr-Thr-Pro-Lys (SEQ ID N°20) ; Glu-Glu-Gly-Asn-Glu-Thr-Glu-Pro-Lys (SEQ ID N°21) ; Glu-Glu-Gly-Asn-Asp-Thr-Glu-Pro-Lys (SEQ ID N°22) ; Glu-Glu-Gly-Asn-Thr-Thr-Glu-Pro-Lys (SEQ ID N°23) ; Gln-Asp-Ala-His-Lys (SEQ ID N°24) ; Glu-Glu-Lys ; Asp-Asp-Asp-Asp-Lys (SEQ ID N°26) ; Glu-Glu-Ala-Glu-Ala-Trp-His-Trp-Leu-Lys (SEQ ID N°29) ; Glu-Glu-Glu-Ala-Trp-His-Trp-Leu-Lys (SEQ ID N°30) ; Leu-Asp-Gly-Arg-Leu-Glu-Ala-Leu-Lys (SEQ ID N°31) ; Glu-Glu-Leu-Asp-Gly-Arg-Leu-Glu-Ala-Leu-Lys (SEQ ID N°32) ; Glu-Glu-Leu-Asp-Ala-Arg-Leu-Glu-Ala-Leu-Lys (SEQ ID N°33) ; Asp-Thr-His-Lys (SEQ ID N°34) ; Asp-Ala-His-Lys (SEQ ID N°35) ; Glu-His-His-Gly-His-Gly-Lys (SEQ ID N°36) ; Asp-Ser-His-Lys (SEQ ID N°37) ; Gln-Asp-Thr-His-Lys (SEQ ID N°38) ; Glu-Ala-Glu-Ala-Glu-Ala-Gln-Asp-Thr-His-Lys (SEQ ID N°39) ; Glu-Ala-Glu-Ala-Gln-Asp-Thr-His-Lys (SEQ ID N°40) ; Glu-Ala-Gln-Asp-Thr-His-Lys (SEQ ID N°41) ; Trp-His-Trp-Leu-Lys (SEQ ID N°42) ; Glu-Glu-Trp-His-Trp-Leu-Lys (SEQ ID N°43) ; Glu-Glu-Glu-Ala-Trp-His-Trp-Leu-Lys (SEQ ID N°44) ; Glu-Glu-Ala-Glu-Lys (SEQ ID N°45) ; Glu-Glu-Gly-Glu-Pro-Lys (SEQ ID N°46) ; Glu-Ala-Gln-Asp-Ala-His-Lys (SEQ ID N°47) ; Glu-Ala-Glu-Ala-Gln-Asp-Ala-His-Lys (SEQ ID N°48) ; et Glu-Ala-Glu-Ala-Glu-Ala-Gln-Asp-Ala-His-Lys (SEQ ID N°49).

9. Procédé selon la revendication 1, où Xₙ-----X₁ - Lys est choisi dans le groupe consistant en Glu-Glu-Ala-His-Lys (SEQ ID N°1) ; Glu-(Glu-Ala) ₂-His-Lys (SEQ ID N°2) ; Glu-(Glu-Ala) ₃His-Lys (SEQ ID N°3) ; Glu-Glu-Gly-His-Lys (SEQ ID N°4) ; Glu-His-Pro-Lys (SEQ ID N°5) ; Glu-Glu-Gly-Glu-Pro-Lys (SEQ ID N°6) ; Glu-Glu-His-Cys-Lys (SEQ ID N°7) ; Glu-Glu-His-Lys (SEQ ID N°8) ; Glu-His-His-His-Lys (SEQ ID N°9) ; Glu-His-Ala-His-Lys (SEQ ID N°10) ; Glu-Gly-Ala-His-Lys (SEQ ID N°11) ; Glu-His-Gly-His-Gly-Lys (SEQ ID N°12) ; Glu-Glu-Ala-His-Glu-Leu-Lys ( SEQ ID N°13) ; Glu-Glu-Ala-His-Glu-Ile-Lys (SEQ ID N°14) ; Glu-Glu-Ala-His-Glu-Val-Lys (SEQ ID N°15) ; Glu-Glu-Ala-His-Glu-Met-Lys (SEQ ID N°16) ; Glu-Glu-Ala-His-Glu-Phe-Lys (SEQ ID N°17) ; Glu-Glu-Ala-His-Glu-Tyr-Lys (SEQ ID N°18) ; Glu-Glu-Ala-His-Glu-Trp-Lys (SEQ ID N°19) ; Gln-Asp-Ala-His-Lys (SEQ ID N°24) ; Glu-Glu-Glu-Ala-Trp-His-Trp-Leu-Lys (SEQ ID N°30) ; Asp-Thr-His-Lys (SEQ ID N°34) ; Glu-His-His-Gly-His-Gly-Lys (SEQ ID N°36) ; Asp-Ser-His-Lys (SEQ ID N°37) ; Gln-Asp-Thr-His-Lys(SEQ ID N°38) ; Glu-Ala-Glu-Ala-Glu-Ala-Gln-Asp-Thr-His-Lys (SEQ ID N°39) ; Glu-Ala-Glu-Ala-Gln-Asp-Thr-His-Lys (SEQ ID N°40) ; Glu-Ala-Gln-Asp-Thr-His-Lys (SEQ ID N°41) ; Trp-His-Trp-Leu-Lys (SEQ ID N°42) ; Glu-Glu-Trp-His-Trp-Leu-Lys (SEQ ID N°43) ; Glu-Glu-Glu-Ala-Trp-His-Trp-Leu-Lys (SEQ ID N°44) ; Glu-Ala-Gln-Asp-Ala-His-Lys (SEQ ID N°47) ; Glu-Ala-Glu-Ala-Gln-Asp-Ala-His-Lys (SEQ ID N°48) ; et Glu-Ala-Glu-Ala-Glu-Ala-Gln-Asp-Ala-His-Lys (SEQ ID N°49).

10. Procédé selon la revendication 1, où Xₙ-----X₁ - Lys est Glu-Glu-Ala-Glu-Lys, SEQ ID N°45 ; Glu-Glu-Gly-Glu-Pro-Lys, SEQ ID N°46 ou Glu-Glu-Lys.

11. Procédé selon la revendication 1, où Xₙ-----X₁ - Lys est choisi dans le groupe consistant en Glu-Glu-Ala-Glu-Ala-Trp-His-Trp-Leu-Lys (SEQ ID N°29) ; Glu-Glu-Glu-Ala-Trp-His-Trp-Leu-Lys (SEQ ID N°30) ; Leu-Asp-Gly-Arg-Leu-Glu-Ala-Leu-Lys (SEQ ID N°31) ; Glu-Glu-Leu-Asp-Gly-Arg-Leu-Glu-Ala-Leu-Lys (SEQ ID N°32) ; Glu-Glu-Leu-Asp-Ala-Arg-Leu-Glu-Ala-Leu-Lys (SEQ ID N°33) ; Glu-Glu-Trp-His-Trp-Leu-Lys (SEQ ID N°43) ; et Glu-Glu-Glu-Ala-Glu-Ala-Trp-His-Trp-Leu-Lys (SEQ ID N°44).

12. Procédé selon la revendication 1, où l'extension N-terminale est choisie dans le groupe consistant en Glu-Glu-Gly-Asn-Thr-Thr-Pro-Lys (SEQ ID N°20) ; Glu-Glu-Gly-Asn-Glu-Thr-Glu-Pro-Lys (SEQ ID N°21) ; Glu-Glu-Gly-Asn-Asp-Thr-Glu-Pro-Lys ( SEQ ID N°22) ; et Glu-Glu-Gly-Asn-Thr-Thr-Glu-Pro-Lys (SEQ ID N°23).

13. Procédé selon la revendication 1, où le clivage enzymatique de l'étape (iv) est réalisé par utilisation d'une lysine-endopeptidase telle que la protéase 1 d'*Achromobacter lyticus.*

14. Procédé selon la revendication 1, où le polypeptide souhaité appartient à la famille de peptides GRF (facteur de libération de l'hormone de croissance), ayant un His ou un Tyr sur la position N-terminale et Ser, Ala ou Gly sur la position la plus proche.

15. Procédé selon la revendication 14, où le polypeptide souhaité est le GLP-1 ou le GLP-2 ou un analogue ou un dérivé du GLP-1 ou du GLP-2.

16. Procédé selon la revendication 15, où l'analogue du GLP-1 est ARg³⁴GLP-1 ₍₇₋₃₇₎ acylé en position Lys²⁶.

17. Procédé selon la revendication 1, où l'étape d'acylation (iii) est effectuée dans un solvant organique ou dans un mélange d'eau et d'un solvant organique en présence d'ions métalliques.

18. Procédé selon la revendication 1, où l'étape d'acylation est réalisée à un pH compris entre 7 et 12.
